# EUROPEAN PATENT APPLICATION

(11) **EP 1 048 669 A1**
(43) Date of publication of application: **02.11.2000**
(21) Application number: 98961372.4
(22) Date of filing: 16.12.1998
(51) Int. Cl.: C07D 477/20, A61K 31/40

(54) **CARBAPENEM DERIVATIVES**

(30) Priority: 16.12.1997 JP 462297; 09.09.1998 JP 27672098
(71) Applicant: BANYU PHARMACEUTICAL CO., LTD., Chuo-ku, Tokyo 103-8416 (JP)
(72) Inventor: NAKANO, Masato, Ibaraki 315-0052 (JP); KIYONAGA, Hideo, Banyu Pharm. Co., Ltd., Tsukuba-shi, Ibaraki 300-2611 (JP); IMAMURA, Hideaki, Banyu Pharm. Co., Ltd., Tsukuba-shi, Ibaraki 300-2611 (JP); MITSUYA, Aya, Banyu Pharm. Co., Ltd., Tsukuba-shi, Ibaraki 300-2611 (JP); SATO, Hiroki, Banyu Pharmaceutical Co., Ltd., Okazaki-shi, Aichi 444-0858 (JP); SUGIMOTO, Yuichi, Banyu Pharmaceutical Co., Ltd., Tsukuba-shi, Ibaraki 300-2611 (JP); SAKURABA, Shunji, Banyu Pharmaceutical Co., Ltd., Tsukuba-shi, Ibaraki 300-2611 (JP); NAKAGAWA, Susumu, 32-1, Aza Gomae, Nukata-gun, Aichi 444-0114 (JP); FUKATSU, Hiroshi, Banyu Pharmaceutical Co., Ltd., Meguro-ku, Tokyo 153-8680 (JP); USHIJIMA, Ryosuke, Banyu Pharmaceutical Co., Ltd., Okazaki-shi, Aichi 444-0858 (JP); HASHIZUME, Terutaka, Banyu Pharm. Co., Ltd., Tsukuba-shi, Ibaraki 300-2611 (JP); NAGANO, Rie, Banyu Pharmaceutical Co., Ltd., Tsukuba-shi, Ibaraki 30-2611 (JP)
(74) Representative: Votier, Sidney David
(86) International application number: JP9805694
(87) International publication number: WO9931106

(57) **Abstract**

Compounds represented by general formula (I); a process for producing the same; and utilization thereof as antimicrobial agents and metallo-β-lactamase inhibitors, wherein R¹ represents hydrogen or lower alkyl; R² represents hydrogen, an ester residue, etc.; R³ and R⁴ are the same or different and each represents hydrogen, halogeno, etc.; R⁵ and R⁶ are the same or different and each represents hydrogen, lower alkoxy, etc., or lower alkyl optionally having one or two substituents selected from the group consisting of carbamoyl, carboxy and hydroxy; group (a) represents aryl selected from the group consisting of benzene and naphthalene rings, etc.; and Y represents a single bond, linear C₁₋₄ alkylene, etc. (provided that the case where R³ and R⁴ are hydrogen, (a) is benzene, Y is methylene and R⁵ and R⁶ are hydrogen is excluded).

## Description

### TECHNICAL FIELD

This invention relates to novel carbapenem compounds (7-oxo-1-azabicyclo[3.2.0]hepta-2-ene-2-carboxylic acid), an antibacterial agent and metallo β-lactamase inhibitor containing said compounds as active ingredient, and a method for producing said compounds.

### BACKGROUND ART

Imipenem, i.e. the carbapenem class antibiotic developed by Merck & Co., Inc. [(5R,6S)-3-[2-(formimidoylamino)-ethylthio]-6-[(R)-1-hydroxyethyl]-7-oxo-1-azabicyclo[3.2.0]hepta-2-ene-2-carboxylic acid monohydrate; J. Med. Chem., Vol.22, Page 1435 (1979)), has potent antibacterial activity against various bacterial species and excellent resistance bacterial β-lactamases. Imipenem exhibits 2-4 times enhanced antibacterial activity compared to the parent antibiotic, thienamycin especially against Pseudomonas aeruginosa.

In the recent years, however, methicillin-resistant Staphylococcus aureus (hereinafter, simply referred to as MRSA), methicillin-resistant coagulase negative staphylococci (hereinafter, referred to as MRCNS) and antibiotic-resistant Pseudomonas aeruginosa are frequently isolated from immunocompromised patients as difficult-to-treat organisms to incur an important social problem. Particularly, vancomycin is used as a potent anti-MRSA agent, but it is not an ideal drug because of its nephrotoxicity, and the increasing resistance of MRSA and MRCNS to vancomycin are becoming great problems from the clinical point of view.

Since imipenem is decomposed by renal dehydropeptidase (hereinafter, referred to as DHP-1), it cannot be used for urinary tract infection treatment and is found to cause nephrotoxicity. Thus, imipenem cannot be administered alone and it requires co-administration with a DHP-1 inhibitor such as cilastatin [J. Antimicrobial Chemother., Vol. 12, (Suppl D) Page 1 (1983)].

Further, imipenem has another problem that it has an adverse effect on the central nervous system in addition to nephrotoxicity. Accordingly, it is intensely desired to develop an agent which has improved antibacterial activity against the difficult-to-treat organisms, especially MRSA and MRCNS, sufficient stability to DHP-1, and reduced nephrotoxicity and toxicity in the central nervous system.

On the other hand, β-lactamase is one of the important resistance factors to β-lactam antibiotics and is classified into classes A, B, C and D according to the primary amino acid sequence thereof. Class B β-lactamase is a metallo enzyme containing zinc in the active center thereof. From the viewpoint of line generation it is different from other classes (classes A, C and D) of serine enzymes having a serine residue in the active center thereof.

Class B β-Lactamase is generally called carbapenemase because it is to hydrolyze carbapenems. Recently, however, it is recommended to call it metallo-β-lactamase, since it hydrolyzes cephalosporins and penicillins more efficiently and therefore such a name causes misunderstanding [B. A. Ramussen et. al., Antimicrob. Agents Chemother., Vol. 41, No. 2, Page 223-232 (1997) and D. M. Livermore, ASM News, Vol. 59, No. 3, Page 129-135 (1993)].

There are two types of metallo-β-lactamases, one of which is chromosome-mediated enzyme and the other is plasmid-mediated enzyme. As enzyme-producing bacterial species belonging to the former type, for examples, Stenotrophomonas (Xantomonas) maltophilia, Bacteroides fragilis, Aeromonas hydrophila, Bacillus cereus etc. can be referred to, and these are known from relatively many years ago.

On the other hand, the latter type of ones produce transferable plasmid-mediated enzyme. In the recent clinics, these bacterial species are isolated in the clinics as difficult-to-treat organisms. Those which have been found to belong to this type are, for example, Pseudomonas aeruginosa, Serratia marcescence, Klebsiella pneumoniae, Bacteroides fragillis, etc. [M. Watanabe, et al., Antimicrob. Agent Chemother., Vol. 35, No. 1, Pages 147-151 (1991) and H. Ito, et al., Antimicrob. Agent Chemother., Vol. 39, No. 4, Pages 824-829 (1995)].

The enzyme produced by these bacteria is roughly the same as the plasmid-mediated enzymes produced by the enteric bacteria in molecular level and enzymological properties, and considered to be distributed among the enteric bacteria. Since this enzyme (metallo-β-lactamase) becomes resistant not only to carbapenems but also to other β-lactam antibiotics, it attracts attention as an important factor conferring resistance to β-lactam antibiotics in the future.

Among the existing β-lactamase inhibitors, clavulanate and sulbactam are effective against class A β-lactamase and class D β-lactamase, and combined agents comprising amoxicillin and cefoperazone with respective inhibitors have been developed. As an inhibitor having expanded spectrum covering class C β-lactamase, a combined agent of tazobactam with piperacillin has been developed, though no sufficient effect has been reported till today. Nothing is known about the inhibitor against class B β-lactamase at the present time, and researches and studies are being energetically conducted to find an inhibitor for class B β-lactamase.

The characteristic feature of this invention consists in a partial structure of the following steric configuration at the 2-position of the carbapenem skeleton: 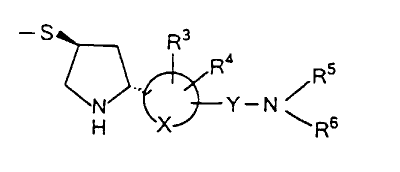 Carbapenems having such a partial structure are novel compounds not found in literature, and there is known no prior art disclosing or suggesting this invention.

An important structural characteristic feature of the carbapenems of this invention consists in the steric configuration of the 2- and 5-positions of the pyrrolidine ring. Due to the asymmetric carbon atoms present in the 2-position and 5-position of the pyrrolidine ring, there can exist four isomers. The compounds of this invention are characterized by having a trans relative configuration and (2R,4S) absolute configuration. Although there have so far been known a number of carbapenums which have a 2,4-disubstituted pyrrolidine ring on the 2-position of carbapenem skeleton with limited configuration of the substituent, and no compound having a (2R, 4S) pyrrolidinylthio group with a trans relative configuration as seen in this invention has been reported in literature, except for only one as mentioned later.

As prior arts closest to this invention, WO9523150 (hereinafter, referred to as Document A) and JP-A-63-170379 (hereinafter, referred to as Document B) can be referred to.

Document A discloses a compound having a substituent A2-W-A3-Ar on the pyrrolidinyithio group.

Document A (from Page 9, Line 26 of the specification), however, mentions that the suitable configuration of the pyrrolidinyl group is (2'S,4'S) and (2'R,4'R) with a cis relative configuration, without referring to a trans relative configuration of the substituents of the 2- and 5-positons of the pyrrolidine ring constituting the characteristic feature of this invention, nor suggesting it at all.

Further, Document A also describes a compound having a 5-[(4-aminomethyl)phenyl]-pyrrolidin-3-ylthio group on the 2-position of the carbapenem skeleton (Example 139) which may have a trans relative configuration. However, there are no description about the stereochemistry of pyrrolidine ring of those diastereomers nor antibacterial activity thereof, only physicochemical data of two diastereomers are mentioned.

In Document B, there is described, carbapenems having a pyrrolidinylthio group substituted with a heterocyclic substituent R⁴ optionally having an appropriate substituent on the 2-position of the carbapenem skeleton.

However, Document B does not refer to the stereoisomers due to the asymmetric carbon atoms of pyrrolidine ring at all, only giving a general description (from Page 7, Left Upper Column, Line 12 of the specification) that, in the objective compound (I) and the starting compound mentioned later, one or more sterecisomers such as optical isomers exist due to the asymmetric carbon atoms, and such isomers are also included in the scope of the invention. That is, there is given no suggestion as to a trans relative configuration of the substituents of the 2- and 5-positions of pyrrolidine ring which constitutes a characteristic feature of this invention. Further, the compounds referred to in Document B as examples are limited to those having a (2S,4S)-substituted pyrrolidinylthio group with a cis relative configuration. This means that, at the present time, specialists in the art do not recognize the advantage given by a trans relative configuration between the substituents of the 2- and 5-positions of pyrrolidine ring.

Further, as the compounds having an inhibitory activity to class B β-lactamase, the phenatidine derivative isolated from the fermentation culture of Streptomyces sp. [M. L. Gilpin et al., J. Antibiotics, Vol. 48, No. 10, Pages 1081-1085] and the derivatives of pyrrolidine and thiazole derivatives (WO9710225) are the only compounds known up to today, and these compounds are entirely different from the compounds of this invention in structure. Further, WO9723483 (hereinafter, referred to as Document C) describes carbapenems having a β-lactamase inhibitory activity. But, Document C makes no concrete disclosure as to the class of the β-lactamase against which its inhibitory activity is exhibited. Accordingly, it is entirely unknown that the compounds of this invention have an inhibitory activity against the class B β-lactamase.

### DISCLOSURE OF THE INVENTION

With the aim of providing novel carbapenems having a broad antibacterial spectrum and an inhibitory activity against class B β-lactamase and showing a resistance to DHP-1, the present inventors have conducted extensive studies.

As its result, it has been found that the carbapenems of this invention having a group represented by the following general formula on the 2-position of a carbapenem skeleton are novel compounds not found in literature and have an intense antibacterial activity against a wide variety of gram-positive bacteria and gram-negative bacteria and an inhibitory activity against class B β-lactamase.

General formula: 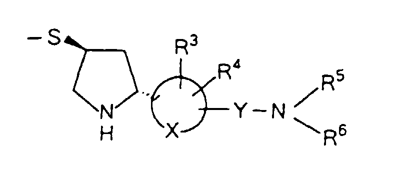 wherein R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group,

R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxy group,
the group represented by the formula 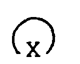 represents an aryl group selected from the group consisting of a benzene ring and a naphthalene ring, a heteroaromatic ring selected from the group consisting of a pyrrole ring, a furan ring and a thiophene ring, or a 5- to 8-membered cycloalkyl group, and
Y represents a single bond, or a 3- to 6-membered cycloalkylene group which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group, or a straight or branched chain alkylene group having 1 to 4 carbon atoms,
provided that a case where R³ and R⁴ both represent a hydrogen atom, the group of the formula 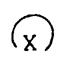 is a benzene ring, Y is a methylene group and R⁵ and R⁶ both represent a hydrogen atom is excepted.

Based on these findings, this invention has been accomplished.

The present inventors have further found that the compounds of the following general formula [I] are not readily susceptible to metabolism in living bodies due to the configuration of the 2- and 5-positions of the pyrrolidine ring with a trans relative configuration, and much higher stability than the corresponding compounds having a cis relative configuration. Based on these findings, this invention has been accomplished.

This invention relates to a compound represented by the following general formula [I]: 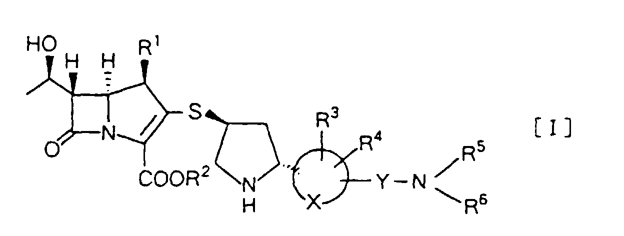 wherein R¹ represents a hydrogen atom or a lower alkyl group; R² represents a hydrogen atom, an ester residue or an alkali metal; R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group; R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxyl group;
the group represented by the formula 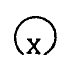 represents an aryl group selected from the group consisting of a benzene ring and a naphthalene ring, a heteroaromatic ring selected from the group consisting of a pyrrole ring, a furan ring and a thiophene ring, or a 5- to 8-membered cycloalkyl group; and
Y represents a single bond, or a 3- to 6-membered cycloalkylene group which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group, or a straight or branched chain alkylene group having 1 to 4 carbon atoms;
provided that a case where the group represented by the formula 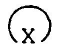 is a benzene ring, Y is a methylene group, and R⁵ and R⁶ both represent a hydrogen atom is excepted;
and to a method for producing said compound; and to the use of said compound as an antibacterial agent and a metallo-β-lactamase inhibitor.

Next, the symbol marks and terms used in this specification will be explained.

The compounds of this invention have the following fundamental structure: 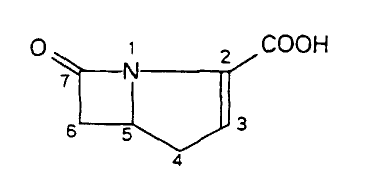 and systematically called 7-oxo-1-azabicyclo[3.2.0]-hepta-2-ene-2-carboxylic acids. In this specification, carbapenem compound numbers widely used conventionally are added to these compounds for simplification, and their fundamental structure (shown below) is referred to as 1-carbapen-2-em-3-carboxylic acid.

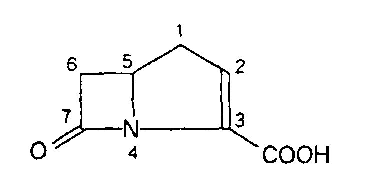

This invention includes optical isomers and stereoisomers due to the asymmetric carbon atoms of 1-, 5-, 6- and 8-positions of carbapenem skeleton. Among these isomers, preferable compounds are the compounds of (5R,6S,8R) configuration in which a 5R,6S configuration (5,6-trans) is retained and the carbon atom of 8-position has an R-configuration, such as the steric configuration of Thienamycin. Further, in a case where the 1-position has a methyl group, those taking a configuration of (1R,5S,6S,8R) are also preferable.

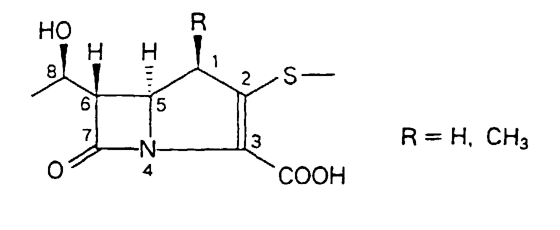

The term "lower alkyl group" means a straight or branched chain alkyl group having 1 to 6 carbon atoms, such as a methyl group, an ethyl group, a propyl group, an isopropyl group, a butyl group, a sec-butyl group, a t-butyl group, a pentyl group, a hexyl group and the like, among which preferable are a methyl group, an ethyl group, a t-butyl group and the like.

The term "cyclo(lower alkyl) group" means a cyclic alkyl group having 3 to 6 carbon atoms, such as a cyclopropyl group, a cyclobutyl group, a cyclopentyl group, a cyclohexyl group and the like, among which preferable are a cyclopropyl group, a cyclobutyl group and the like.

The term "lower alkenyl group" means a straight or branched chain alkenyl group having 2 to 6 carbon atoms, such as a vinyl group, a 1-propenyl group, an allyl group, an isopropenyl group, a 1-butenyl group, a 3-butenyl group, a 1,3-butanedienyl group, a 2-pentenyl group, a 4-pentenyl group, a 1-hexenyl group, a 3-hexenyl group, a 5-hexenyl group and the like, among which preferable are a 1-propenyl group, an allyl group, an isopropenyl group, a 1-butenyl group and the like.

The term "lower alkynyl group" means a straight or branched chain alkynyl group having 2 to 6 carbon atoms, such as a 2-propynyl group, a 2-butynyl group, a 3-butynyl group, a 2-pentynyl group and the like, among which preferable are a 2-propynyl group, a 2-butynyl group and the like.

The term "lower alkoxy group" means a straight or branched chain alkyloxy group having 1 to 6 carbon atoms, such as a methoxy group, an ethoxy group, a propoxy group, an isopropoxy group, a butoxy group, a sec-butoxy group, a t-butoxy group, a pentyloxy group, a hexyloxy group and the like, among which preferable are a methoxy group, an ethoxy group, a t-butoxy group and the like.

The term "lower alkylthio group" means a straight or branched chain alkylthio group having 1 to 6 carbon atoms, such as a methylthio group, an ethylthio group, a propylthio group, an isopropylthio group, a butylthio group, a sec-butylthio group, a t-butylthio group, a pentylthio group, a hexylthio group and the like, among which preferable are a methylthio group, an ethylthio group, a t-butylthio group and the like.

The term "amino-substituted lower alkyl carbonyl group" means an amino-substituted, straight or branched chain alkylcarbonyl group having 1 to 6 carbon atoms, such as an aminomethylcarbonyl group, a 2-aminoethylcarbonyl group, a 3-aminopropylcarbonyl group, a 2-amino-1-methylethylcarbonyl group, a 4-aminobutylcarbonyl group, a 3-amino-2-methylpropylcarbonyl group, a 2-amino-1,1-dimethylethylcarbonyl group and the like, among which preferable are an aminomethylcarbonyl group, a 2-aminoethylcarbonyl group, a 2-amino-1,1-dimethylethylcarbonyl group and the like.

The term "lower alkylimidoyl group" means a straight or branched chain alkylimidoyl group having 1 to 6 carbon atoms, such as a methylimidoyl group, an ethylimidoyl group, a propylimidoyl group, an isopropylimidoyl group, a butylimidoyl group, a sec-butylimidoyl group, a t-butylimdoyl group, a pentylimidoyl group, a hexylimidoyl group and the like, among which preferable are a methylimidoyl group, an ethylimidoyl group, a t-butylimidoyl group and the like.

The term "aryl group" means, for example, a benzene ring, a naphthalene ring and the like, among which preferable are a benzene ring and a naphthalene ring.

As the "straight or branched chain alkylene group having 1 to 4 carbon atoms", a methylene group, an ethylene group, a methylmethylene group, a propylene group, a dimethylmethylene group, a butylene group and the like can be referred to, among which preferable are a methylene group, an ethylene group, a propylene group and the like.

As the "3- to 6-membered cycloalkylene group", a cyclopropylene group, a cyclobutylene group, a cyclopentylene group, a cyclohexylene group and the like can be referred to, among which preferable are a cyclopropylene group, a cyclobutylene group and the like.

The term "ester residue" means, for example, alkanoyloxymethyl groups such as an acetoxymethyl group, a pivaloyloxymethyl group and the like, alkoxycarbonyloxyalkyl groups such as a 1-(ethoxycarbonyloxy) ethyl group and the like, a phthalidyl group, a (5-substituted-2-oxo-1,3-dioxol-4-yl) methyl group such as a (5-methyl-2-oxo-1,3-dioxol-4-yl) methyl group and the like, etc.

As the "alkali metal", for example, alkali metals such as sodium, potassium and the like can be referred to, among which preferable is sodium.

As the protecting group for a carboxyl group, for example, there can be referred to lower alkyl groups such as a methyl group, an ethyl group, a propyl group, an isopropyl group, a t-butyl group and the like; halo-substituted lower alkyl groups such as a 2,2,2-trichloroethyl group, a 2,2,2-trifluoroethyl group and the like; lower alkanoyloxyalkyl groups such as an acetoxymethyl group, a propionyloxymethyl group, a pivaloyloxymethyl group, a 1-acetoxyethyl group, a 1-propionyloxyethyl group and the like; lower alkoxycarbonyloxyalkyl groups such as a 1-(methoxycarbonyloxy) ethyl group, a 1-(ethoxycarbonyloxy) ethyl group, a 1-(isopropoxycarbonyloxy) ethyl group and the like; lower alkenyl groups such as a 2-propenyl group, a 2-chloro-2-propenyl group, a 3-methoxycarbonyl-2-propenyl group, a 2-methyl-2-propenyl group, a 2-butenyl group, a cinnamyl group and the like; aralkyl groups such as a benzyl group, a p-methoxybenzyl group, a 3,4-dimethoxybenzyl group, an o-nitrobenzyl group, a p-nitrobenzyl group, a benzhydryl group, a bis(p-methoxyphenyl) methyl group and the like; (5-substituted-2-oxo-1,3-dioxol-4-yl) methyl groups such as a (5-methyl-2-oxo-1,3-dioxol-4-yl) methyl group and the like; lower alkylsilyl groups such as a trimethylsilyl group, a t-butyldimethylsilyl group and the like; an indanyl group; a phthalidyl group; a methoxymethyl group; and the like. Among these protecting groups for a carboxyl group, particularly preferable are a 2-propenyl group, a p-nitrobenzyl group, a p-methoxybenzyl group, a benzhydryl group, a t-butyldimethylsilyl group and the like.

As the protecting group for a hydroxyl group, for example, there can be referred to lower alkylsilyl groups such as a trimethylsilyl group, a t-butyldimethylsilyl group and the like; lower alkoxymethyl groups such as a methoxymethyl group, a 2-methoxyethoxymethyl group and the like; a tetra-hydropyranyl group; aralkyl groups such as a benzyl group, a p-methoxybenzyl group, a 2,4-dimethoxybenzyl group, an o-nitrobenzyl group, a p-nitrobenzyl group, a trityl group and the like; acyl groups such as a formyl group, an acetyl group and the like; lower alkoxycarbonyl groups such as a t-butoxycarbonyl group, a 2-iodoethoxycarbonyl group, a 2,2,2-trichloroethoxycarbonyl group and the like; alkenyloxycarbonyl groups such as a 2-propenyloxycarbonyl group, a 2-chloro-2-propenyloxycarbonyl group, a 3-methoxycarbonyl-2-propenyloxycarbonyl group, a 2-methyl-2-propenyloxycarbonyl group, a 2-butenyloxycarbonyl group, a cinnamyloxycarbonyl group and the like; and aralkyloxycarbonyl groups such as a benzyloxycarbonyl group, a p-methoxybenzyloxycarbonyl group, an o-nitrobenzyloxycarbonyl group, a p-nitrobenzyloxycarbonyl group and the like. Among these protecting groups, particularly preferable are a 2-propenyloxycarbonyl group, a p-nitrobenzyloxycarbonyl group, a t-butyldimethylsilyl group and the like.

As the protecting group for an amino group, for example, there can be referred to aralkylidene groups such as a benzylidene group, a p-chlorobenzilidene group, a p-nitrobenzylidene group, a salicylidene group, an α-naphthylidene group, a β-naphthylidene group and the like; aralkyl groups such as a benzyl group, a p-methoxybenzyl group, a 3,4-dimethoxybenzyl group, an o-nitrobenzyl group, a p-nitrobenzyl group, a benzhydryl group, a bis(p-methoxyphenyl) methyl group, a trityl group and the like; lower alkanoyl groups such as a formyl group, an acetyl group, a propionyl group, a butyryl group, an oxalyl group, a succinyl group, a pivaloyl group and the like; halo-substituted lower alkanoyl groups such as a chloroacetyl group, a dichloroacetyl group, a trichloroacetyl group, a trifluoroacetyl group and the like; arylalkanoyl groups such as a phenylacetyl group, a phenoxyacetyl group and the like; lower alkoxycarbonyl groups such as a methoxycarbonyl group, an ethoxycarbonyl group, a propoxycarbonyl group, a t-butoxycarbonyl group and the like; halo-substituted lower alkoxycarbonyl groups such as a 2-iodoethoxycarbonyl group, a 2,2,2-trichloroethoxycarbonyl group and the like; alkenyloxycarbonyl groups such as a 2-propenyloxycarbonyl group, a 2-chloro-2-propenyloxycarbonyl group, a 3-methoxycarbonyl-2-propenyloxycarbonyl group, a 2-methyl-2-propenyloxycarbonyl group, a 2-butenyloxycarbonyl group, a cinnamyloxycarbonyl group and the like; aralkyloxycarbonyl groups such as a benzyloxycarbonyl group, an o-nitrobenzyloxycarbonyl group, a p-nitrobenzyloxycarbonyl group, a phenethyloxycarbonyl group and the like; and lower alkylsilyl groups such as a trimethylsilyl group, a t-butyldimethylsilyl group and the like. Among these protecting groups, particularly preferable are a 2-propenyloxycarbonyl group, a t-butoxycarbonyl group, a p-nitrobenzyloxycarbonyl group and the like.
R¹ represents a hydrogen atom or a lower alkyl group.
R² represents a hydrogen atom, an ester residue or an alkali metal.
R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group, among which preferable are a hydrogen atom, a halogen atom, a lower alkyl group, a carbamoyl group and a hydroxy group and particularly preferable are a hydrogen atom, a halogen atom and a lower alkyl group.
R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxy group, among which preferable are a hydrogen atom, a lower alkoxy group, an amino-substituted lower alkyl carbonyl group, a sulfamoyl group, a formimidoyl group, a lower alkylimidoyl group, an amidino group and a lower alkyl group which may have one or two substituents selected from the group consisting of a carbamoyl group and a hydroxy group, and particularly preferable are a hydrogen atom, a lower alkoxy group, an amino-substituted lower alkyl carbonyl group, a sulfamoyl group, a lower alkylimidoyl group, an amidino group and a lower alkyl group which may have a substituent selected from the group consisting of a carbamoyl group and a hydroxy group.

The group represented by the formula 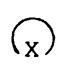 represents an aryl group selected from the group consisting of a benzene ring and a naphthalene ring, a heteroaromatic ring selected from the group consisting of a pyrrole ring, a furan ring and a thiophene ring, or a 5- to 8-membered cycloalkyl group, among which preferable are an aryl group selected from the group consisting of a benzene ring and a naphthalene ring, a heteroaromatic ring selected from the group consisting of a furan ring and a thiophene ring and a 5- or 6-membered cycloalkyl group, and particularly preferable are an aryl group selected from the group consisting of a benzene ring and a naphthalene ring, a heteroaromatic ring selected from the group consisting of a furan ring and a thiophene ring and a cyclohexyl group.

Y represents a single bond, or a 3- to 6-membered cycloalkylene group which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group, or a straight or branched chain alkylene group having 1 to 4 carbon atoms.

Next, the compound of the general formula [I] will be explained more concretely.

Among the compounds represented by the general formula [I], preferable compounds are those represented by the following general formula [I-a]: 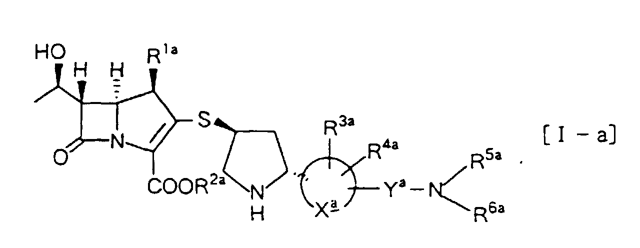 wherein R^{1a} represents a hydrogen atom or a lower alkyl group; R^{2a} represents a hydrogen atom, an ester residue or an alkali metal; R^{3a} and R^{4a}, identical or different, represent a hydrogen atom, a halogen atom, a lower alkyl group, a carbamoyl group or a hydroxy group; R^{5a} and R^{6a}, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group and a hydroxy group;
the group represented by the formula 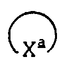 represents an aryl group selected from the group consisting of a benzene ring and a naphthalene ring, a heteroaromatic group selected from the group consisting of a furan ring and a thiophene ring, or a 5- or 6-membered cycloalkyl group; and Y^{a} represents a single bond, or a 3- to 6-membered cycloalkylene group which may have one or two substituents selected from the group consisting of a lower alkenyl group, a lower alkylthio group, a carbamoyl group, a hydroxy group, a sulfamoyl group and a thiocarbamoyl group, or a straight or branched chain alkylene group having 1 to 4 carbon atoms, provided that a case where R^{3a} and R^{4a} represent a hydrogen atom, the group represented by the formula 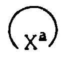 is a benzene ring, Yₐ is a methylene group and R^{5a} and R^{6a} represent a hydrogen atom is excepted; and
particularly preferable compounds are those represented by the following general formula [I-b]: 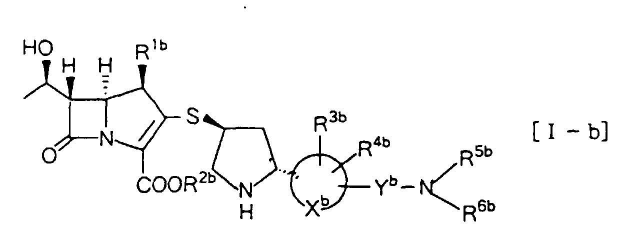 wherein R^{1b} represents a hydrogen atom or a lower alkyl group; R^{2b} represents a hydrogen atom, an ester residue or an alkali metal; R^{3b} and R^{4b}, identical or different, represent a hydrogen atom, a halogen atom or a lower alkyl group; R^{5b} and R^{6b}, identical or different, represent a hydrogen atom, a lower alkoxy group, an amino-substituted lower alkyl carbonyl group, a sulfamoyl group, a lower alkylimidoyl group, an amidino group, or a lower alkyl group which may have a substituent selected from the group consisting of a carbamoyl group and a hydroxy group; the group represented by the formula 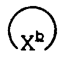 represents an aryl group selected from the group consisting of a benzene ring and a naphthalene ring, a heteroaromatic group selected from the group consisting of a furan ring and a thiophene ring or a cyclohexyl group; and Y^{b} represents a single bond, or a 3- to 6-membered cycloalkylene group which may have a substituent selected from the group consisting of a lower alkenyl group, a lower alkylthio group, a carbamoyl group, a hydroxy group and a sulfamoyl group, or a straight or branched chain alkylene group having 1 to 4 carbon atoms; provided that a case where R^{3b} and R^{4b} both represent a hydrogen atom, the group represented by the formula 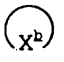 is a benzene ring, Y_{b} represents a methylene group and R^{5b} and R^{6b} both represent a hydrogen atom is excepted.

The pharmacologically acceptable salt of the compound of the general formula [I] means a salt which is conventionally used and pharmacologically acceptable. As examples thereof, there can be referred to those compounds in which the carboxyl group of the 3-position of carbapenem skeleton or the basic or acidic residue on the side chain of the 2-position is converted to a salt form.

As the salt formed by adding a base to the carboxyl group or the acidic residue, there can be referred to the alkali metal salts in which R² is an alkali metal such as a sodium salt, a potassium salt and the like; alkaline earth metal salts such as a calcium salt, a magnesium salt and the like; an ammonium salt; a trimethylamine salt and a triethylamine salt; aliphatic amine salts such as a dicyclohexylamine salt, an ethanolamine salt, a diethanolamine salt, a triethanolamine salt, a procaine salt and the like; aralkylamine salts such as a N,N'-dibenzylethylenediamine salt and the like; heteroaromatic amine salts such as a pyridine salt, a picoline salt, a quinoline salt, an isoquinoline salt and the like; quaternary ammonium salts such as a tetramethylammonium salt, a tetraethylammonium salt, a benzyltrimethylammonium salt, a benzyltriethylammonium salt, a benzyltributylammonium salt, a methyltrioctylammonium salt, a tetrabutylammonium salt and the like; and salts of basic amino acids such as an alginic acid salt and a lysine salt and the like.

As the salt formed by adding an acid to the base on the side chain of the 2-position, there can be referred to inorganic acid salts such as hydrochloride, sulfate, nitrate, phosphate, carbonate, hydrogen carbonate, perchlorate and the like; organic acid salts such as acetate, propionate, lactate, maleate, fumarate, tartrate, malate, citrate, ascorbate and the like; sulfonic acid salts such as methanesulfonate, isethionate, benzenesulfonate, p-toluenesulfonate and the like; and salts of acidic amino acids such as aspartate, glutarate and the like.

As the pharmacologically acceptable nontoxic ester of the compound of the general formula [I], the conventionally used and pharmacologically acceptable esters formed from the 3-carboxyl group of carbapenem skeleton can be referred to. That is, esters formed from the ester residue of R² can be referred to.

Next, the method for producing the compounds of this invention will be explained.

A compound represented by the general formula [II]: 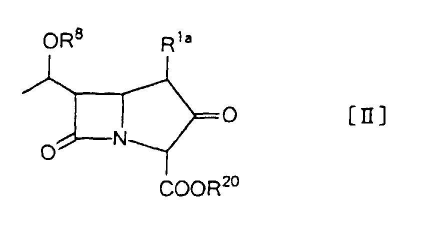 wherein R^{1a} is a hydrogen atom or a lower alkyl group, R⁸ is a hydrogen atom or a protecting group for a hydroxyl group, and R²⁰ is a hydrogen atom or a protecting group for a carboxyl group, is reacted with an activating reagent in an inert organic solvent in the presence of a base to derive the compound [II] into a reactive derivative [II']: 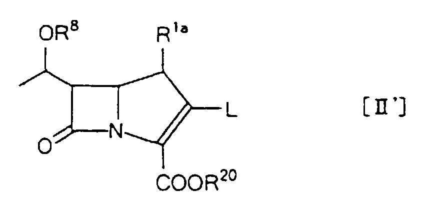 wherein L represents a group which can be eliminated, and R^{1a}, R⁸ and R²⁰ are as defined above.

As the inert organic solvents which can be used in the above reaction, diethyl ether, tetrahydrofuran , dioxane, benzene, toluene, chlorobenzene, methylene chloride, chloroform, carbon tetrachloride, dichloroethane, trichloroethylene, acetone, ethyl acetate, acetonitrile, N,N-dimethylformamide, hexamethyl phosphorotriamide and mixtures thereof can be referred to, among which acetonitrile, benzene and the like are particularly preferable.

As the bases usable in the above reaction, there can be referred to tertiary aliphatic amines such as trimethylamine, triethylamine, N,N-diisopropylethylamine, N-methylmorpholine, N-methylpyrrolidine, N-methylpiperidine, N,N-dimethylaniline, 1,8-diazabicyclo-[5.4.0]undeca-7-ene (DBU), 1,5-diazabicyclo[4.3.0]nona-5-ene (DBN) and the like; and aromatic amines such as pyridine, 4-dimethylaminopyridine, picoline, lutidine, quinoline, isoquinoline and the like. Among these bases, N,N-diisopropylethylamine, triethylamine and the like are particularly preferable.

As the activating reagents usable in the above reaction, there can be referred to acid anhydrides such as trifluoroacetic anhydride, methanesulfonic anhydride, trilfluoromethanesulfonic anhydride, p-toluenesulfonic anhydride and the like; and acid chlorides such as methanesulfonyl chloride, p-toluenesulfonyl chloride, diphenyl chlorophosphate and the like. Among these activating reagents, diphenyl chlorophosphate is particularly preferable.

In the general formula [II'], L means a group which can be eliminated. Examples thereof include a trifluoroacetoxy group, a methanesulfonyloxy group, a trifluoromethanesulfonyloxy group, a p-toluenesulfonyloxy group, a diphenoxyphosphoryloxy group and the like, among which particularly preferable is a diphenoxyphosphoryloxy group.

In the reaction, the base is used in an amount of 1-3 mol and preferably 1-1.5 mol, and the activating reagent is used in an amount of 1-1.2 mol, both per mol of the compound of the general formula [II].

The reaction is carried out at a temperature of -40°C to 50°C and preferably -20°C to 20°C. Usually, the reaction reaches completion quantitatively in 0.5-3 hours.

After completion of the reaction, the reaction mixture is worked up in the conventional manner to obtain the reactive derivative [II']of the general formula [II] in a quantitative yield.

The reaction between the compound of the general formula [II] or its reactive derivative [II'] and the compound represented by the general formula [III]: 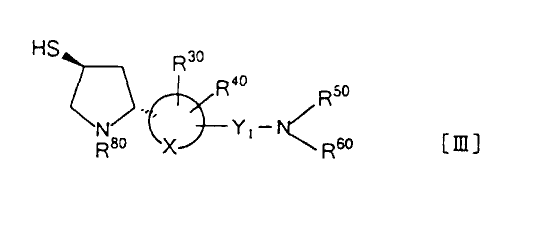 wherein R³⁰ and R⁴⁰, identical or different, represent a hydrogen atom, a halogen atom, a lower alkyl group, a lower alkenyl group, a lower alkynyl group, a lower alkoxy group, a lower alkylthio group, a carbamoyl group, a carboxyl group which may be protected, a cyano group, a hydroxy group which may be protected, a nitro group, a sulfamoyl group, a sulfamoylamino group or an ureido group; R⁵⁰ and R⁶⁰, identical or different, represent a hydrogen atom, a lower alkoxy group, an amino-substituted lower alkyl carbonyl group which may be protected, a sulfamoyl group, a formimidoyl group, a lower alkylimidoyl group, an amidino group, or a lower alkyl group which may have one or two substituents selected from the group consisting of a carbamoyl group, a carboxyl group which may be protected and a hydroxy group which may be protected;
the group represented by the formula 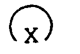 represents an aryl group selected from the group consisting of a benzene ring and a naphthalene ring, a heteroaromatic group selected from the group consisting of a pyrrole ring, a furan ring and a thiophene ring, or a 5- to 8-membered cycloalkyl group;
Y₁ represents a single bond, or a 3- to 6-membered cycloalkylene group which may have one or two substituents selected from the group consisting of a halogen atom, a lower alkenyl group, a cyclo(lower alkyl) group, a lower alkoxy group, a lower alkylthio group, a carbamoyl group, a carboxyl group which may be protected, a cyano group, a hydroxy group which may be protected, a nitro group, a sulfamoyl group, a sulfamoylamino group, a thiocarbamoyl group and an ureido group, or a straight or branched chain alkylene group having 1 to 4 carbon atoms; and
R⁸⁰ represents a hydrogen atom or a protecting group for an imino group;
provided that a case where R³⁰ and R⁴⁰ both represent a hydrogen atom, the group represented by the formula 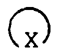 is a benzene ring, Y₁ is a methylene ring, and R⁵⁰ and R⁶⁰ both represent a hydrogen atom is excepted;
is carried out by the use of the above-mentioned inert organic solvent and base, whereby a compound represented by the following general formula [IV]: 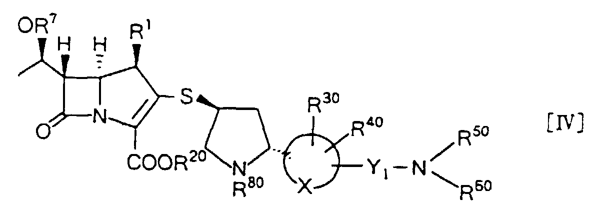 wherein R¹, R⁷, R²⁰, R³⁰, R⁴⁰, R⁵⁰, R⁶⁰, R⁸⁰, the group represented by the formula 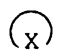 and Y₁ are as defined above, can be produced.

The reaction is carried out by using a salt such as lithium chloride or the like in an amount of 1-5 mol and preferably 1.5-2 mol and the compound of the general formula [III] in an amount of 1-3 mol and preferably 1.2-1.5 mol, per mol of the compound of the general formula [II] or its reactive derivative [II'], at a temperature of -40°C to 60°C and preferably 0°C to 30°. Usually, the reaction reaches completion in 3-30 hours.

It is also possible to produce the compound of the general formula [IV] from the compound of the general formula [II] in one step. That is, without isolating the reactive derivative [II'] derived from the compound of the general formula [II], the compound of the general formula [III] is subjected to the same reaction as mentioned above in the same reaction system as above, whereby the compound of the general formula [IV] can be produced efficiently.

After completion of the reaction, the reaction mixture is conventionally worked-up to obtain a crude product of the compound [IV], which may be subjected to elimination of the protecting group without purification. Preferably, the crude product [IV] thus obtained is purified by crystallization, silica gel column chromatography or the like.

The compound [IV] thus obtained is subjected to an appropriate combination of elimination reactions of the protecting groups of a hydroxyl group, an amino group and a carboxyl group depending upon the case, and, if desired, the compound thus obtained is further converted to a pharmacologically acceptable salt or a non-toxic ester, whereby a compound of the general formula [I] can be produced.

The protecting group may be removed in various manners depending on the kind of the protecting group. That is, it is carried out by, for example, solvolysis, chemical reduction or hydrogenation in the usual way.

In a case where, in the general formula [IV], the protecting group of a hydroxyl group and/or an amino group is an aralkyloxycarbonyl group such as a benzyloxycarbonyl group, a p-nitrobenzyloxycarbonyl group or the like and the protecting group of a carboxyl group is an aralkyl group such as a benzyl group, a p-nitrobenzyl group, a benzhydryl group or the like, the protecting group can be eliminated by a catalytic hydrogenation using a platinum catalyst such as platinum oxide, platinum wire, platinum black or the like or a palladium catalyst such as palladium black, palladium oxide, palladium-carbon, palladium hydroxide-carbon or the like.

As the solvent which can be used in the catalytic hydrogenation, there can be referred to, for example, methanol, ethanol, tetrahydrofuran, dioxane, acetic acid and the like and mixtures of these organic solvents and water or a buffer solution such as phosphate buffer or the like.

The catalytic hydrogenation is carried out under a 1-4 hydrogen atmosphere, at a temperature of 0-50°C. The reaction is completed in 0.5-24 hours and preferably 5-15 hours.

In a case where, in the general formula [IV], the protecting group of a hydroxyl group and/or an amino group is, for example, an allyloxycarbonyl group and the protecting group of a carboxyl group is, for example, an allyl group, the protecting group can be eliminated by reacting a palladium complex catalyst in an inert organic solvent containing an allyl group-scavenger [the method of W. McCombie, cf. J. Org. Chem., Vol. 47, Pages 587-590 (1982) and the method of F. Guibé et al., cf. ibid., Vol. 52, Pages 4984-4993 (1987)].

As the solvent used in the reaction, water, acetone, diethyl ether, tetrahydrofuran, dioxane, ethyl acetate, acetonitrile, methylene chloride, chloroform and the like and mixtures thereof can be referred to.

As the preferable palladium catalyst usable in this reaction, there can be referred to, for example, palladium-carbon, palladium hydroxide-carbon, palladium (II) chloride, palladium (II) acetate, tetrakis(triphenylphosphine) palladium (0), tetrakis(triphenoxyphosphine) palladium (0), tetrakis(triethoxyphosphine) palladium (0), bis[ethylenebis(diphenylphosphine)] palladium (0), tetrakis[tri(2-furyl)-phosphine] palladium (0), bis(triphenylphosphine) palladium (II) chloride, bis(triphenylphosphine) palladium (II) acetate and the like.

As the allyl group-scavenger, there can be referred to, for example, dimedone, formic acid, acetic acid, ammonium formate, sodium formate, sodium 2-ethylhexanoate, potassium 2-ethylhexanoate, pyrrolidine, piperidine, tributyltin hydride and the like.

The reaction is carried out with 0.01-0.5 mol of catalyst and 1-6 mol of an allyl group-scavenger, per mol of the compound of the general formula [IV], at a temperature of -10°C to 50°C and preferably at 0°C to 30°C. Usually, the reaction is completed in 0.5-3 hours.

In a case where, in the general formula [IV], the protecting group of a hydroxyl group and/or an amino group is an o-nitrobenzyloxycarbonyl group and the protecting group of a carboxyl group is an o-nitrobenzyl group, the protecting groups can be eliminated by a photo-reaction [cf. the method of Amit et al., J. Org. Chem., Vol. 39, Page 192-196 (1974)].

After eliminating the protecting group, the compound of the general formula [I] can be isolated by a conventional treatment such as a column chromatography using silica gel or an adsorbing resin, or a liophilization or crystallization.

In a case where, in the compound of the general formula [IV], the protecting group of the carboxyl group of the 3-position is a lower alkanoyloxyalkyl group such as an acetoxymethyl group, a pivaloyloxymethyl group or the like, or a methoxymethyl group, an indanyl group, a phthalidyl group or the like, such esters are hydrolyzed in the living body physiologically. Accordingly, it is possible to administer the compound directly to human being and animals without eliminating the protecting group.

The compound of the general formula [I] can be converted to a pharmacologically acceptable salt or ester in the conventional manner.

The starting compound represented by the general formula [II] can be produced according to the method of Salzmann et al. [J. Am. Chem. Soc., Vol. 102, pages 6161-6163 (1981)] in a case where R² is hydrogen atom, and according to the method of Shih et al. [Heterocycles, Vol. 21, Pages 29-40 (1984)] in a case where R² is a methyl group, or according to similar methods.

The compound of the general formula [III] can be produced according to the descriptions of Referential Examples 1 and 2.

The compound of this invention can be used as a medical drug containing it as an active ingredient, and particularly as an antibacterial agent and a metallo-β-lactamase inhibitor. In such medical drugs and particularly antibacterial agent and metallo-β-lactamase, the compound of this invention may be a compound represented by the general formula [I']: 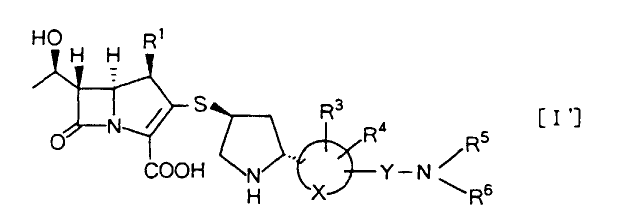 wherein R¹, R³, R⁴, R⁵, R⁶, the group represented by the formula 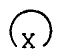 and Y are as defined above, or a pharmacologically acceptable ester or salt thereof at the 3-carboxyl group of the carbapenem skeleton, or an inner salt formed between the basic or acidic residue present on the side chain of the 2-position of said compound, ester or salt and the 3-carboxyl group, and particularly a compound represented by the general formula [I]. Herein, the pharmacologically acceptable ester and salt include those mentioned above.

The compound of this invention exhibits an intense antibacterial activity against various gram-positive bacteria and gram-negative bacteria.

In order to demonstrate the usefulness of the compound of this invention concretely, its in vitro antibacterial activity was measured according to the following agar plate dilution method [the standard method of Japanese Chemotherapeutic Society:
Chemotherapy, Vol. 29, Pages 76-79 (1981)], using the compounds of Example 139A of Document A and Example 10-3) of Document B, both having a cis relative configuration, as control compounds. A Mueller Hinton agar (MH agar) was inoculated with a platinum loop quantity (the quantity of inoculated bacterium: 10⁶ CFU/ml) of each test bacterial strain which had been cultured overnight in Mueller Hinton broth. The culture medium contained an antibacterial agent at a varied concentration. After a culture at 37°C for 16 hours, the minimum growth inhibitory concentration (MIC: µg/ml) of the compound of this invention was measured. When MRSA was used as the test strain, a medium containing 2% sodium chloride was used as the test medium in accordance with the above-mentioned agar plate dilution method, and the minimum growth inhibitory concentration (MIC: µg/ml) of the compound of this invention was measured under culture conditions of 35°C, 48 hours. The results are summarized in Table 1.

**Table 1**

| Minimum growth inhibitory concentrations (MIC: µg/ml) | | | | | |
|---|---|---|---|---|---|
| Strain tested | Compound | | | | |
| | Ex.14 | Ex.15 | Ex.32 | Ex.139A in Document A | Ex.10-3) in Document B |
| E. coli NIHJ JC2 | 0.025 | 0.025 | 0.025 | 0.05 | 0.05 |
| P. aeruginosa IFO-3445 | 0.1 | 0.2 | 0.39 | 0.78 | 3.13 |
| MRSA BB6294 | 3.13 | 3.13 | 3.13 | 6.25 | 25 |

The compound of this invention has an excellent antibacterial activity against a broad spectrum of gram-positive bacteria and gram-negative bacteria, and are useful as an antibacterial agent used for treatment and prevention of the bacterial infectious diseases of human being caused by these pathogenie bacteria. As the typical causal bacteria sensible to the antibacterial agent of this invention, there can be referred to, for example, bacterial strains belonging to Genus Staphylococcus, Genus Enteroococcus, Genus Escherichia, Genus Enterobactor, Genus Klebsiella, Genus Serratia, Genus Proteus, Genus Pseudomonas, etc.

The compound of this invention also exhibits an intense metallo-β-lactamase inhibitory activity.

### 〈β-Lactamase inhibition test〉

Metallo-β-lactamase encoded by plasmid pMS350 originated from Pseudomonas aeruginosa GN17203 was purified and used for this test. The enzyme previously gave a single protein band on SDS/PAGE (specific activity: 70 U/mg protein). The enzymatic activity (U) was measured by reacting it with 100 µM of imipenem dissolved in 10 mM 3-morpholinopropanesulfonate buffer (pH 7.0) (hereinafter referred to as MOPS buffer) containing 100 µM zinc chloride, and the quantity of enzyme capable of hydrolyzing 1 µM of imipenem per one minute at a reaction temperature of 30°C was defined as 1 U.

As the screening substrate of this test, Nitrocefin®, OXOID) was used. In each well of a 96 well plate, 1 µl of a test agent dissolved in 10 mM MOPS buffer and 25 µl of metallo-β-lactamase preparation (enzymatic activity: 3-6 mU/ml) were mixed, and then 75 µl of 50 µg/ml Nitrocefin® was added and mixed to create a final substrate concentration of 37.5 µg/ml. The enzymic reaction was carried out at 30°C for 15 minutes and followed by measuring the quantity of hydrolyzed Nitrocefin™ at a wavelength of 492 nm by means of a microplate reader. In this screening system, the operation was carried out in the presence of Zn²⁺ (100 µM) in order to eliminate the inhibitory effect due to chelating action. The concentration of test reagent exhibiting 50% inhibition was calculated as IC₅₀, using a MOPS buffer in place of inhibitor as a control. The results are listed in Table 2.

**Table 2**

| Metallo-β-lactamase inhibitory activities | |
|---|---|
| Test compound | IC₅₀ (µM) |
| Compound of Referential. Ex. 1 | < 0.1 |
| Compound 1-1 of Document C | > 10 |
| Compound 3-4 of Document C | > 10 |

In cases where the compound of this invention is usable as a metallo-β-lactamase inhibitor, its pharmacologically acceptable salt is also usable. As typical example of such pharmacologically acceptable salt, for example, salts formed with alkali metals such as sodium, potassium and the like can be referred to.

Further, the compound of this invention is markedly improved in the symptoms of the central nervous system and the nephrotoxicity, so that it is quite low in toxicity and high in safety. Hereinunder, various toxicity tests of the compound of this invention on the central nervous system will be described.

First of all, a toxicity test on the central nervous system is described.

### 〈Toxicity test on the central nervous system〉

A chronic guide cannula was inserted into the right ventricle of the brain of 7-weeks old male SD rats (n = 5) and immobilized with a dental resin. After a recovering period of one week, a test compound which had been dissolved in physiological salt solution and adjusted to about pH 7 was administered to the right ventricle of the brain at a dosage of 200-400 µg/10 µl/head by means of a microsyringe. The compound of Example 139A of Document A was used as a control compound. After the administration, behavior of the animal was visually examined in an observing cage for thirty minutes. The results are summarized in Table 3.

**Table 3**

| Central nerve toxicity test in rats | | | |
|---|---|---|---|
| Test compound | Dosage (µg/rat) | Number of convulsed cases | Number of dead cases |
| Ex. 1 | 400 | 0/5 | 0/5 |
| Ex. 14 | 200 | 0/5 | 0/5 |
| Ex. 15 | 200 | 0/5 | 0/5 |
| Compound of Ex.139A in Document A | 100 | 4/5 | 1/5 |
| Compound of Ex.139B in Document A | 100 | 4/5 | 1/5 |

The results shown above demonstrate that the compound of this invention exercises no toxicity on the central nervous system at all and gives no case of death.

Next, acute toxicity test of the compound of this invention will be described.

### 〈Acute toxicity test〉

The compound of Example 24 was used as a representative compound. According to "Explanation Book for GLP Standard and Toxicity Test Guideline" (supervised by Department of Examination, Bureau of Pharmacy, Ministry of Health and Welfare; published by Yakuji Shimpou-sha), a test compound was diluted with distilled water for injection use until the agent concentration reached 60.75 mg/ml and once administered intravenously to the tail vein of 6-weeks old male ICR mice at an administration rate of 1 ml/min. After the administration, the state of death and the general motility were observed over five days. The results are summarized in Table 4.

**Table 4**

| Acute toxicity on mouse | | |
|---|---|---|
| Dosage (mg/kg) | Number of alive animals | Motility and general symptoms |
| 1,500 | 5/5 | No abnormality |
| 1,000 | 5/5 | No abnormality |
| 500 | 2/2 | No abnormality |

Next, in vivo stability test of the compound of this invention will be mentioned below.

### 〈In vivo stability test〉

The compound of Example 1 was used as a representative compound, and the compound of Example 139B of Document A was used as a comparative compound. A test agent (10 mg/kg) was intravenously administered to 4-years old male rhesus monkey (body weight 5.48 kg) over a period of 5 minutes. The urine was collected over a period of 0-1 hour and over a period of 1-2 hours, both after the administration. The urine samples were stored at an ice-cooled temperature. A TLC plate (silica gel 60F-254, manufactured by Merck Co.) which had been activated just before use at 120°C for 30 minutes and spotted with 4 µl of each urine sample, was developed with a solvent in a closed vessel (developing solvent: CH₃CN/H₂O/CH₃COOH/25% NH₄OH = 45:30:6:2; pH 5.3). After completion of the development, the TLC plate was taken out of the vessel, and dried. An AM-1 medium (manufactured by Difco Co.) containing a test strain (B. subtilis ATCC 12432) was contacted with the silica gel surface of the TLC plate at ambient temperature for 30 minutes. After stripping off the TLC plate, the medium was cultured at 37°C overnight. R_{f} value of a clear zone formed by the growth inhibition of test strain was determined. As control, the test compound was dissolved in 10 mmol MOPS buffer (pH 7) at the concentration of 250 µg/ml, and 2 µl of this solution was spotted on a TLC plate. The results were as shown in Table 5.

**Table 5**

| In vivo stability test | | | |
|---|---|---|---|
| | R_{f} value | | |
| | Control | Accumulated urine during 0-1 hr after administration | Accumulated urine during 1-2 hrs after administration |
| Compound of Ex.1 | 0.40 | 0.40 | 0.40 |
| Active metabolite of the compound of Ex.1 | Undetected | Undetected | Undetected |
| Compound of Ex.139B in Document A | 0.47 | 0.47 | Undetected |
| Active metabolite of the compound of Ex.139B in Document A | Undetected | 0.82 | 0.82 |

It is apparent from the results presented above that the compound of this invention having a trans configuration between the 2- and 5-substituents of pyrrolidine ring has much higher stability than does the comparative compound in which the configuration between the 2- and 5-substituents of pyrrolidine ring is cis, and the compound of this invention is not susceptible to metabolism, and has highly safety profile.

When the compound of this invention is used as an antibacterial agent and a metallo-β-lactamase inhibitor, it can also be used in the form of a pharmacologically acceptable salt thereof. As typical examples of the pharmacologically acceptable salt, salts with alkali metals such as sodium, potassium and the like can be referred to.

A pharmacologically acceptable salt of the compound of this invention can be produced according to an appropriate combination of the methods conventionally used in organic synthesis. More concretely saying, it can be produced by, for example, neutralizing a solution of a compound of this invention in its free form with a solution of alkali.

As the administration form at the time of using the compound of this invention as an antibacterial agent and a metallo-β-lactamase inhibitor, a variety of forms can be adopted. The forms adoptable include oral preparations such as tablets, capsule, powder, granules, liquid preparation and the like and sterilized liquid non-oral preparations such as solution, suspension and the like.

Although a solid preparation can directly be made into a form of tablet, capsule, granule or powder, it is also possible to produce it after adding suitable additives. As such additives, there can be referred to sugars such as lactose, glucose and the like; starches such as corn, wheat, rice and the like; fatty acids such as stearic acid and the like; inorganic salts such as sodium metasilicate, magnesium aluminate, anhydrous calcium phosphate and the like; synthetic polymers such as polyvinylpyrrolidone, polyalkylene glycol and the like; fatty acid salts such as calcium stearate, magnesium stearate and the like; alcohols such as stearyl alcohol, benzyl alcohol and the like; synthetic cellulose derivatives such as methyl cellulose, carboxymethyl cellulose, ethyl cellulose, hydropropyl methyl cellulose and the like; water; gelatin; talc; vegetable oils; gum arabic; and other conventional additives.

These solid preparations such as tablet, capsule, granule, powder, etc. can contain the active ingredient generally in an amount of 0.1-100% by weight and preferably in an amount of 5-100% by weight.

A liquid preparation can be produced as a form of liquid suspension, syrup, injection and the like by the use of a proper additive conventionally used in liquid preparations such as water, alcohols, oils derived from vegetable oils, namely soybean oil, peanut oil, sesame oil or the like.

Particularly in the case of non-oral administration, namely intramuscular, intravenous or subcutaneous administration, suitable solvents are as follows: distilled water for injection, aqueous solution of lidocaine hydrochloride for intramuscular injection, physiological salt solution, aqueous solution of glucose, ethanol, liquid for use in the intravenous injection such as aqueous solutions of citric acid, sodium citrate and the like, solutions of electrolytes for intravenous drip, intravenous injection, etc., and mixed solutions thereof.

These injections may be not only those previously formed by dissolution but also those having a form of dry powder or its mixture with appropriate additives which can be made into a solution just before use. These injections can contain the active ingredient usually in an amount of 0.1-10% by weight and preferably in an amount of 1-5% by weight.

The orally administered liquid preparations such as suspension, syrup and the like can contain the active ingredient in an amount of 0.5-10% by weight.

The actually preferred dosage of the compound of this invention can be appropriately increased or decreased depending on the compound, the composition prepared therefrom, the frequency of use, the site, and the state of illness. For example, the dosage per day per one adult is 10 to 500 mg in the oral administration and 10 to 100 mg per day in the case of non-oral administration and preferably intravenous injection. Although frequency of administration varies depending on the method of administration and symptom, a single administration or a multiple administration in 2 to 5 portions may be adopted.

If desired, it is also possible to administer the compound of this invention in combination with a DHP-I inhibitor such as cilastatin [sodium (Z)-7-(L-amino-2-carboxyethylthio)-2-(2,2-dimethylcyclopropane carboxamido)-2-heptenoate], etc. [JP-A-56-81518; European Patent No. 28,778; J. Med. Chem., Vol. 30, Page 1074 (1987)].

### EXAMPLES AND REFERENTIAL EXAMPLES

This invention is explained more concretely below by referring to examples, referential examples and formulation examples. This invention is by no means limited by these examples.

In the thin layer chromatography referred to in the examples and referential examples, Silicagel 60F₂₄₅ (Merck) was used and a UV detector was used for detection. The silica gel for the column chromatography was Wakogel® C-300 (Wako Junyaku), and the silica gel for reversed phase column chromatography was LC-SORB® SP-B-ODS (Chemco) or YMC-GEL® ODS-AQ 120-S50 (YMC). As high-performance liquid chromatography, JASCO 800 series (JASCO) was used. In the NMR spectroscopic measurement, an internal standard was tetramethylsilane (TMS) when dimethyl sulfoxide d-6 or chloroform d-1 was used as a solvent and 2,2-dimethyl-2-silapentane-5-sulfonate (DSS) was used as an internal standard when the deuterium oxide was used as a solvent. The measurement was carried out with XL-200 (200 MHz, Varian) spectrometer. The δ values were expressed in terms of ppm.

The abbreviations in the NMR data have the following meanings:
s: singlet
d: doublet
dd: double doublet
quint: quintet
m: multiplet
br: broad
J: coupling constant
Hz: hertz
CDCl₃: chloroform-d
D₂O: deuterium oxide

The abbreviations used in the reaction schemes, etc. have the following meanings:
Ac: acetyl group
Et: ethyl group
n-Bu: n-butyl group
Bz: benzyl group
n-Pr: n-propyl group,
i-Pr: isopropyl group
Me: methyl group
Ph: phenyl group
PNB: p-nitrobenzyl group
POM: pivaloyloxymethyl group
Py: pyridyl group
TEA: triethylamine

### Example 1

### (1R,5S,6S)-2-[(3S,5R)-5-[4-N-methylaminomethylphenyl]-pyrrolidin-3-ylthio]-6-[(R)-1-hydroxyethyl]-1-methyl-1-carbapen-2-em-3-carboxylic acid hydrochloride

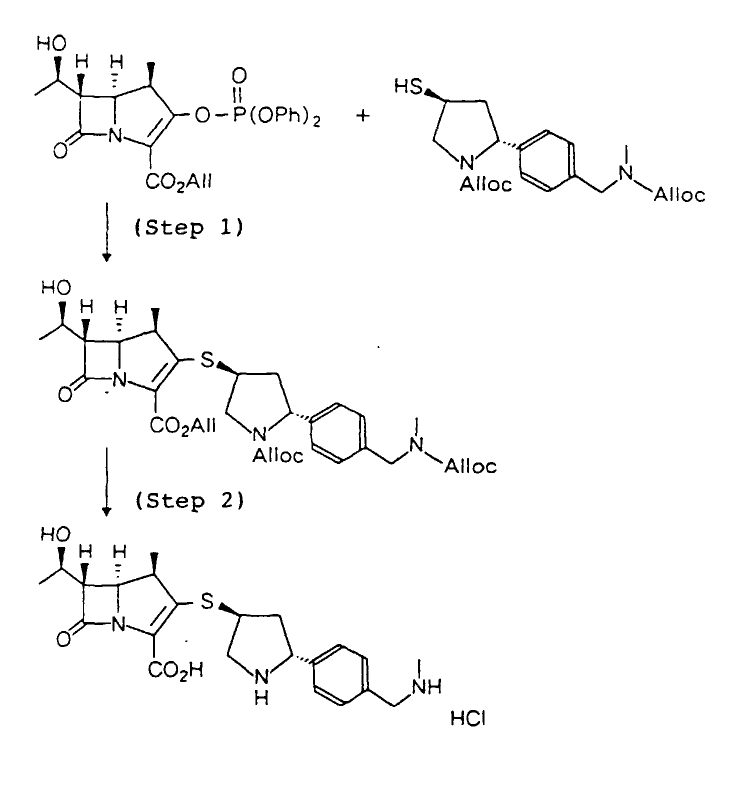

### (Step 1)

Under a stream of nitrogen, 1N NaOH (214 ml, 214 mmol) was added at an ice-cooled temperature to a solution of (2R,4S)-4-acetylthio-1-allyloxycarbonyl-2-(4-N-allyloxycarbonyl-N-methylaminomethyl)-phenylpyrrolidine (83.93 g, 194 mmol) in methanol (850 ml). The resulting mixture was stirred at the same temperature as above for 30 minutes. The mixture was neutralized with 1N HCl (214 ml, 214 mmol), and then ethyl acetate and water were added. The organic layer was washed successively with water and brine and dried on anhydrous sodium sulfate, and the solution was evapolated under reduced pressure. As a result, a crude thiol compound was obtained. Under a stream of nitrogen, a solution of the thiol obtained above in acetonitrile (1,000 ml) and diisopropylamine (hereinafter, referred to as DIPA) (42.5 ml, 252 mmol) were added to a solution of allyl (1R,5S,6S)-2-diphenoxyphosphoryloxy-6-[(1R)-1-hydroxyethyl]-1-methyl-1-carbapen-2-em-3-carboxylate (123.25 g, 247 mmol) in acetonitrile (1,000 ml), and the reaction mixture was stirred overnight at 6°C. Ethyl acetate was added to the reaction mixture, and the organic layer was washed successively with water and brine. The organic layer was dried on anhydrous sodium sulfate, and the solution was evaporated under reduced pressure. The residue was purified by silica gel column chromatography (Wakogel® C-300, 1:4 mixture of hexane and ethyl acetate) to obtain allyl (1R,5S,6S)-2-[(3S,5R)-1-allyloxycarbonyl-5-[4-(N-methylallyloxycarbonyl-aminomethyl)phenyl]-pyrrolidin-3-ylthio]-6-[(R)-1-hydroxyethyl]-1-methyl-1-carbapen-2-em-3-carboxylate (66.8 g, yield 53.7%) as a colorless oil.
¹H-NMR (CDCl₃) δ: 1.22 (3H, d, J=7.2Hz), 1.36 (3H, d, J=6.3Hz), 2.26 (1H, m), 2.40 (1H, m), 2.87 (3H, s), 3.23 (1H, dd, J=7.1, 2.6Hz), 3.30 (1H, m), 3.59-3.87 (2H, m), 4.03 (1H, m), 4.24 (2H, m), 4.46 (2H, s), 4.58 (1H, m), 4.64 (2H, m), 4.69 (1H, m), 4.83 (1H, dd, J=13.5, 5.5Hz), 4.93 (1H, m), 5.12 (1H, m), 5.18 - 5.49 (6H, m), 5.93 (3H, m), 7.17 (4H, m)

### (Step 2)

Under a stream of nitrogen, water (9.32 ml), bis(triphenylphosphine) palladium (II) chloride (3.61 g, 5.18 mmol) and tributyltin hydride (100.4 ml, 373.1 mmol) were added to a solution of the compound obtained in Step 1 (66.3 g, 103.6 mmol) in methylene chloride (2,072 ml) at an ice-cooled temperature. After stirring at 0°C for 30 minutes, the resulting mixture was evaporated under reduced pressure. The residue was dissolved in water (1,000 ml) and purified by reversed phase chromatography (eluted with 2,000 ml of 15-20% acetonitrile-H₂O). The objective fractions were collected and evaporated under reduced pressure. The concentrated residue was adjusted to pH 6.0 with 1N HCl, and freeze-dried to obtain the title compound (20.21 g, yield 41.7%).
¹H-NMR (D₂O) δ: 1.22 (3H, d, J=7.3Hz), 1.26 (3H, J=6.3Hz), 2.51 (1H, dd, J=14.0, 7.0Hz), 2.70 (3H, s), 2.74 (1H, m), 3.30- 3.50 (3H, m), 3.85 (1H, dd, J=12.0, 6.0Hz), 4.22 (5H, m), 5.04 (1H, m), 7.53 (4H, s)
IR (KBr) cm⁻¹ : 3373, 2966, 1751, 1587, 1392, 1086

### Example 2

### (1R,5S,6S)-2-[(3S,5R)-5-[4-(1-amino-3-butenyl)phenyl]-pyrrolidin-3-ylthio]-6-[(R)-1-hydroxyethyl]-1-methyl-1-carbapen-2-em-3-carboxylic acid hydrochloride

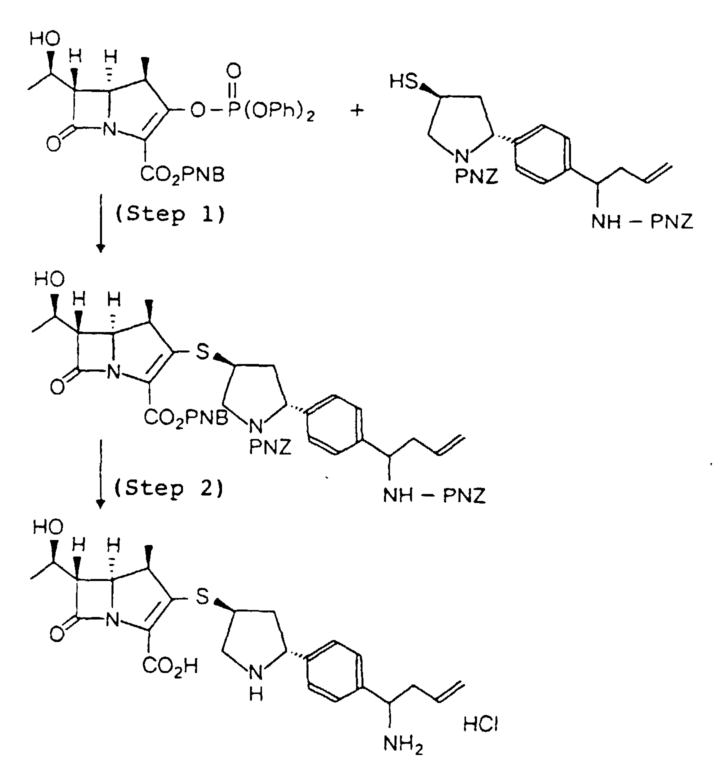

### (Step 1)

Under a stream of nitrogen, NaOMe (1.13 g, 19.84 mmol) was added at an ice-cooled temperature to a solution of (4S)-4-benzoylthio-1-p-nitrobenzyloxycarbonyl-2-[4-(1-p-nitrobenzyloxycarbonylamino-3-butenyl)]-phenylpyrrolidine (7.14 g, 9.92 mmol) in chloroform-methanol mixture (2:1, 300 ml), and the resulting mixture was stirred at the same temperature as above for 4 hours. The mixture was neutralized with 1N HCl (19.8 ml, 19.8 mmol), and chloroform and water were added. The organic layer was washed successively with water and brine and dried on anhydrous sodium sulfate, and the solution was evaporated under reduced pressure to obtain a crude thiol compound. Under a stream of nitrogen, a solution of the thiol obtained above in acetonitrile (150 ml) and DIPA (3.46 ml, 19.84 mmol) were added to a solution of p-nitrobenzyl (1R,5S,6S)-2-diphenxoyphosphoryloxy-6-[(1R)-1-hydroxyethyl]-1-methyl-1-carbapen-2-em-3-carboxylate (5.90 g, 9.92 mmol) in acetonitrile (150 ml), and the reaction mixture was stirred overnight at 6°C. Ethyl acetate was added to the mixture, the organic layer was washed successively with water and brine and dried on anhydrous sodium sulfate, and the solution was evaporated under reduced pressure. The residue was purified by silica gel column chromatography (Wakogel® C-300, hexane-ethyl acetate 1:3 to 0:1) to obtain diastereomer (I), i.e. p-nitrobenzyl (1R,5S,6S)-2-[(3S,5R)-1-p-nitrobenzyloxycarbonyl-5-[4-(1-p-nitrobenzyloxycarbonylamino-3-butenyl)phenyl]- pyrrolidin-3-ylthio]-6-[(R)-1-hydroxyethyl]-1-methyl-1-carbapen-2-em-3-carboxylate (2.69 g, yield 28.5%) as a light yellow-colored oil and diastereomer (II), i.e. p-nitrobenzyl (1R,5S,6S)-2-[(3S,5S)-1-p-nitrobenzyloxycarbonyl-5-[4-(1-p-nitrobenzyloxycarbonyl-amino-3-butenyl)phenyl]pyrrolidin-3-ylthio]-6-[(R)-1-hydroxyethyl]-1-methyl-1-carbapen-2-em-3-carboxylate (2.52 g, yield 26.7%) as a light yellow oil.
¹H-NMR (CDCl₃) δ: 1.26 (3H, t, J=7.1Hz), 1.38 (3H, d, J=6.4Hz), 2.31 (1H, m), 2.38 - 2.61 (3H, m), 3.23 - 3.41 (2H, m), 3.86 (3H, m), 4.04 (1H, m), 4.27 (2H, m), 4.75 - 5.30 (10H, m), 5.49 - 5.76 (2H, m), 6.88 - 8.24 (16H, m)

### (Step 2)

The diastereomer (I) obtained in Step 1 (2.69 g, 2.83 mmol) was dissolved in a mixture of tetrahydrofuran (hereinafter referred to as THF) (160 ml) and phosphate buffer (pH=6.0, 0.5M, 160 ml). At an ice-cooled temperature, zinc powder (32.3 g) was added to the solution obtained above, and stirred at ambient temperature for 5 hours. After filtering off the insoluble matter, the filtrate was concentrated under reduced pressure so that it did not reach dryness, and the additionally deposited insoluble matter was filtered off. The filtrate was purified by reversed phase column chromatography (eluted with 120 ml of 30-50% aqueous methanol). The objective fractions were collected, and the solution was evaporated under reduced pressure. The residue was adjusted to pH 4.5 with 1N HCl, and freeze-dried to obtain the title compound (412 mg, yield 29.5%).
¹H-NMR (D₂O) δ: 1.22 (3H, d, J=7.3Hz), 1.27 (3H, d, J=6.3Hz), 2.54 (1H, m), 2.75 (2H, dd, J=7.1, 7.1Hz), 2.80 (1H, m), 3.37 (1H, m), 3.49 (2H, m), 3.91 (1H, m), 4.23 (3H, m), 4.48 (1H, t, J=7.4Hz), 5.10 (1H, m), 5.18 (2H, m), 5.69 (1H, m), 7.53 (4H, m)
IR (KBr) cm⁻¹ : 1749, 1616, 1396, 1288

### Example 3

### (1R,5S,6S)-2-[(3S,5R)-5-[4-N-(isopropylaminomethyl-phenyl]pyrrolidin-3-ylthio]-6-[(R)-1-hydroxyethyl]-1-methyl-1-carbapen-2-em-3-carboxylic acid hydrochloride

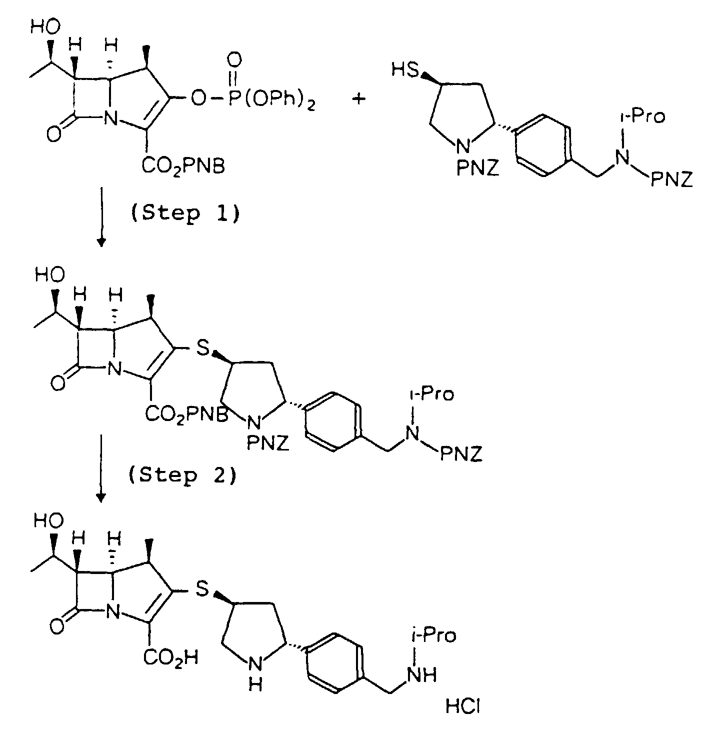

### (Step 1)

Under a stream of nitrogen, 1N NaOH (1.78 ml, 1.78 mmol) was added at an ice-cooled temperature to a solution of (2R,4S)-4-acetylthio-1-p-nitrobenyloxycarbonyl-2-[4-(N-isopropyl-p-nitrobenzyloxycarbonylaminomethyl)phenyl]pyrrolidine (1.16 g, 1.78 mmol) in a mixture of methanol (30 ml) and THF (5 ml). The reaction mixture was stirred at the same temperature as above for 10 minutes. The mixture was neutralized with 1N HCl (2.0 ml, 2.0 mmol), and ethyl acetate and water were added. The organic layer was washed successively with water and brine and dried on anhydrous sodium sulfate, and the solution was evaporated under reduced pressure to obtain a crude thiol compound. Under a stream of nitrogen, a solution of the thiol compound obtained above in acetonitrile (10 ml) and DIPA (0.47 ml, 2.69 mmol) were added to a solution of p-nitrobenzyl (1R,5S,6S)-2-diphenoxyphosphoryloxy-6-[(1R)-1-hydroxyethyl]-1-methyl-1-carbapen-2-em-3-carboxylate (1.06 g, 1.78 mmol) in acetonitrile (40 ml), and the reaction mixture was stirred overnight at 6°C. Ethyl acetate was added to the reaction mixture, the organic layer was washed successively with water and brine and dried on anhydrous sodium sulfate, and the solution was evaporated under reduced pressure. The residue thus obtained was purified by silica gel column chromatography (Walcogel® C-300, ethyl acetate) to obtain p-nitrobenzyl (1R,5S,6S)-6-[(1R)-1-hydroxyethyl]-1-methyl-2-[(3S,5R)-1-p-nitrobenzyloxycarbonyl-5-[4-(N-isopropyl-p-nitrobenzyloxycarbonylaminomethyl)phenyl]pyrrolidin-3-ylthio]-1-carbapen-2-em-carboxylate (1.23 g, yield 71.5%) as a light yellow oil.
¹H-NMR (CDCl₃) δ: 1.16 (3H, d, J=6.7Hz), 1.18 - 1.32 (3H, m), 1.38 (3H, d, J=6.2Hz), 2.28 (1H, m), 2.45 (1H, m), 3.22 - 3.42 (2H, m), 3.68 - 3.92 (2H, m), 4.05 (1H, m), 4.2 - 4.51 (5H, m), 4.88 - 5.42 (6H, m), 5.52 (1H, m), 6.92 (1H, m), 7.08 - 7.61 (7H, m), 7.66 (2H, d, J=8.8Hz), 7.88 - 8.12 (2H, m), 8.22 (4H, d, J=8.8Hz)
IR (KBr) cm⁻¹: 3373, 2966, 1751, 1587, 1392, 1086

### (Step 2)

At an ice-cooled temperature, 10% palladium-carbon (1.23 g) was added to a solution of the compound obtained in Step 1 (1.23 g, 1.27 mmol) in a mixture of 0.2M N-methylmorpholinopropanesulfonate buffer solution (hereinafter, referred to as MOPS buffer) (pH 6.5, 44.5 ml), THF (60 ml) and ethanol (7.5 ml). Then, at ambient temperature, a catalytic reduction was carried out under an atmospheric hydrogen pressure for 16 hours. After filtering off the catalyst, the reaction mixture was evaporated under reduced pressure. The aqueous solution thus obtained was adjusted to pH 6.0 with aqueous solution of sodium hydrogen carbonate, and then washed with ethyl acetate. The aqueous solution was concentrated under reduced pressure, the resulting small quantity of insoluble matter was filtered off, and the filtrate was purified by reversed phase column chromatography (eluted with 50 ml of 40% aqueous methanol), the objective fractions were collected, the solvent was concentrated under reduced pressure, adjusted to pH 6.1 with 1N HCl, and freeze-dried to obtain the title compound (190.6 mg, yield 30.3%).
¹H-NMR (D₂O) δ: 1.22 (3H, d, J=7.3Hz), 1.26 (3H, d, J=6.3Hz), 1.35 (6H, d, J=6.6Hz), 2.52 (1H, m), 2.75 (1H, m), 3.28 - 3.52 (4H, m), 3.88 (1H, m), 4.18 - 4.3 (5H, m), 5.08 (1H, m), 7.48 - 7.6 (4H, m)
IR (KBr) cm⁻¹: 1751, 1650, 1618, 1394

The compounds of Examples 4 to 37 represented by the general formula (I) were produced by the same methods as in Examples 1 to 3.

### Example 4

### (1R,5S,6S)-2-[(3S,5R)-5-[3-aminomethylphenyl]-pyrrolidin-3-ylthio]-6-[(R)-1-hydroxyethyl]-1-methyl-1-carbapen-2-em-3-carboxylic acid hydrochloride

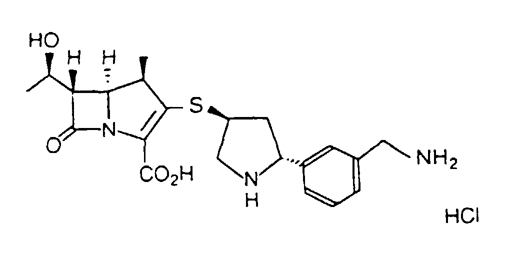

¹H-NMR (D₂O) δ: 1.21 (3H, d, J=7.3Hz), 1.25 (3H, J=6.4Hz), 2.51 (1H, m), 2.74 (1H, m), 3.29 - 3.53 (3H, m), 3.84 (1H, m), 4.15 - 4.30 (5H, m), 5.03 (1H, m), 7.45 - 7.60 (4H, m)
IR (KBr) cm⁻¹: 1749, 1648, 1558, 1540

### Example 5

### (1R,5S,6S)-2-[(3S,5R)-5-[4-(2-aminoethyl)phenyl]pyrrolidin-3-ylthio]-6-[(R)-1-hydroxyethyl]-1-methyl-1-carbapen-2-em-3-carboxylic acid hydrochloride

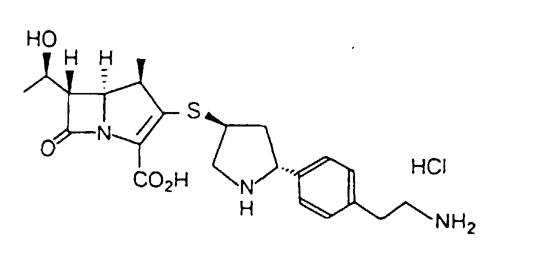

¹H-NMR (D₂O) δ: 1.21 (3H, d, J=7.1Hz), 1.25 (3H, d, J=6.3Hz), 2.47 (1H, m), 2.74 (1H, m), 3.01 (2H, t, J=7.2Hz), 3.26 (2H, t, J=7.2Hz), 3.35 (1H, m), 3.46 (2H, m), 3.83 (1H, m), 4.22 (3H, m), 5.03 (1H, m), 7.37 (2H, d, J=8.3Hz), 7.45 (2H, d, J=8.3Hz)
IR (KBr) cm⁻¹: 1747, 1616, 1153, 721

### Example 6

### (1R,5S,6S)-2-[(3S,5R)-5-[(4-aminomethyl)-3-fluoro)phenyl]-pyrrolidin-3-ylthio]-6-[(R)-1-hydroxyethyl]-1-methyl-1-carbapen-2-em-3-carboxylic acid hydrochloride

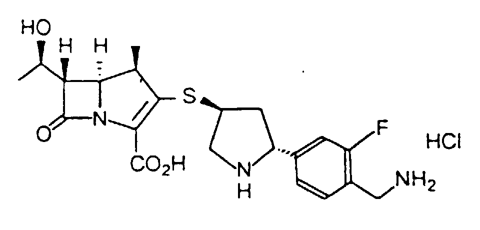

¹H-NMR (CDCl₃) δ: 1.22 (3H, d, J=7.3Hz), 1.26 (3H, d, J=6.3Hz), 2.55 (1H, ddd, J=1.6, 6.7, 12.7Hz), 2.75 (1H, ddd, J=3.3, 7.4, 12.7Hz), 3.34 (1H, dq, J=7.2, 9.3Hz), 3.42 - 3.54 (2H, m), 3.89 (1H, dd, J=5.5, 12.7Hz), 4.21 - 4.26 (5H, m), 5.10 (1H, dd, J=6.4, 11.2Hz), 7.33 - 7.37 (2H, m), 7.54 (1H, m)
IR (KBr) cm⁻¹: 3415, 1749, 1737, 1394, 1079

### Example 7

### (1R,5S,6S)-2-[(3S,5R)-5-[4-aminomethyl-3-methylphenyl]-pyrrolidin-3-ylthio]-6-[(R)-1-hydroxyethyl]-1-methyl-1-carbapen-2-em-3-carboxylic acid hydrochloride

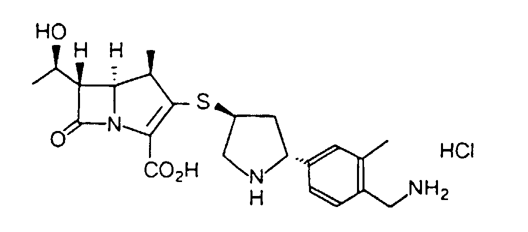

¹H-NMR (D₂O) δ: 1.25 (3H, d, J=7.3Hz), 1.29 (3H, d, J=6.8Hz), 2.41 (3H, s), 2.53 (1H, dd, J=14.1, 6.9Hz), 2.78 (1H, m), 3.39 (1H, m), 3.49 (2H, m), 3.89 (1H, dd, J=12.4, 6.0Hz), 4.25 (2H, s), 4.26 (3H, m), 5.06 (1H, dd, J=11.1, 6.7Hz), 7.40 (3H, m)
IR (KBr) cm⁻¹: 1749, 1635, 1558, 1508, 1396

### Example 8

### (1R,5S,6S)-2-[(3S,5R)-5-[4-N-ethylaminomethylphenyl]-pyrrolidin-3-ylthio]-6-[(R)-1-hydroxyethyl]-1-methyl-1-carbapen-2-em-3-carboxylic acid hydrochloride

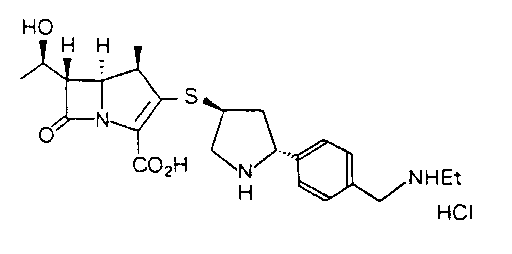

¹H-NMR (D₂O) δ: 1.18 - 1.35 (9H, m), 2.52 (1H, m), 2.75 (1H, m), 3.11 (2H, q, J=7.3Hz), 3.36 (1H, m), 3.42 - 3.52 (2H, m), 3.88 (1H, m), 4.18 - 4.3 (5H, m), 5.08 (1H, m), 7.49 - 7.62 (4H, m)
IR (KBr) cm⁻¹: 1749, 1558, 1394

### Eample 9

### (1R,5S,6S)-2-[(3S,5R)-5-[4-(N-carbamoylmethylaminoethyl)phenyl]pyrrolidin-3-ylthio]-6-[(R)-1-hydroxyethyl]-1-methyl-1-carbapen-2-em-3-carboxylic acid hydrochloride

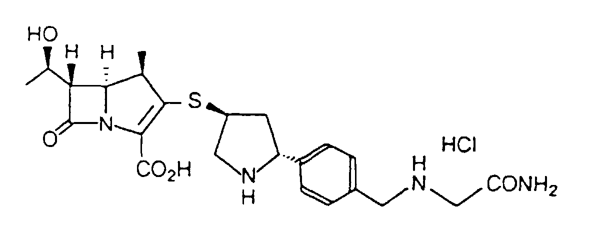

¹H-NMR (D₂O) δ: 1.21 (3H, d, J=6.9Hz), 1.26 (3H, d, J=6.3Hz), 2.40 (1H, m), 2.78 (1H, m), 3.33 - 3.46 (3H, m), 3.74 (1H, dd, J=5.7, 12.5Hz), 4.20 (2H, s), 4.16 - 4.24 (3H, m), 5.08 (1H, m), 6.54 (1H, d, J=3.3Hz), 6.59 (1H, d, J=3.3Hz)
IR (KBr) cm⁻¹: 1753, 1578, 1392, 1259

### Example 10

### (1R,5S,6S)-2-[(3S,5R)-5-(4-N-(2-hydroxyethyl)-aminomethylphenyl]pyrrolidin-3-ylthio]-6-[(R)-1-hydroxyethyl]-1-methyl-1-carhapen-2-em-3-carboxylic acid hydrochloride

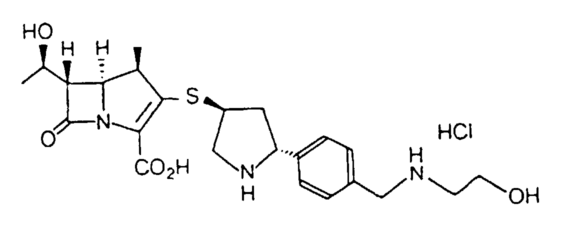

¹H-NMR (D₂O) δ: 1.22 (3H, d, J=7.0Hz), 1.27 (3H, d, J=6.3Hz), 2.51 (1H, m), 2.72 (1H, m), 3.13 - 3.21 (2H, m), 3.28 - 3.51 (3H, m), 3.78 - 3.90 (3H, m), 4.18 - 4.30 (5H, m), 5.03 (1H, m), 7.48 - 7.58 (4H, m)
IR (KBr) cm⁻¹: 1749, 1558, 1394, 1081

### Example 11

### (1R,5S,6S)-2-[(3S,5R)-5-[4-N-(2-aminoethyl-1-oxo)-aminomethylphenyl]pyrrolidin-3-ylthio]-6-[(R)-1-hydroxyethyl]-1-methyl-1-carbapen-2-em-3-carboxylic acid hydrochloride

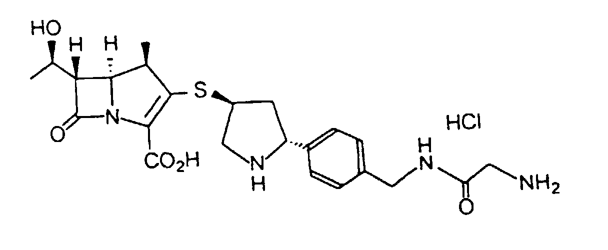

¹H-NMR (D₂O) δ: 1.22 (3H, d, J=7.2Hz), 1.26 (3H, d, J=6.6Hz), 2.49 (1H, m), 2.73 (1H, m), 3.35 (1H, m), 3.46 (2H, m), 3.78 (2H, s), 3.83 (1H, m), 4.21 (3H, m), 4.41 (2H, s), 5.03 (1H, m), 7.38 (2H, d, J=7.9Hz), 7.44 (2H, d, J=7.9Hz)
IR (KBr) cm⁻¹: 1754, 1681, 1575

### Example 12

### (1R,5S,6S)-2-[(3S,5R)-5-[4-methoxyaminomethylphenyl)-pyrrolidin-3-ylthio]-6-[(R)-1-hydroxyethyl]-1-methyl-1-carbapen-2-em-3-carboxylic acid hydrochloride

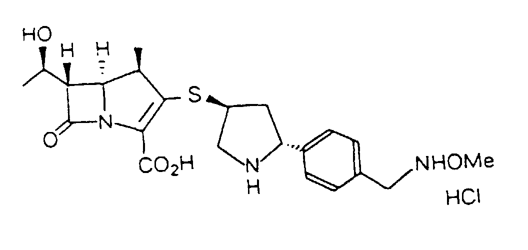

¹H-NMR (D₂O) δ: 1.23 (3H, d, J=7.1Hz), 1.27 (3H, d, J=6.3Hz), 2.52 (1H, m), 2.78 (1H, m), 3.3 - 3.58 (6H, m), 3.88 (1H, m), 4.05 (2H, s), 4.2 - 4.3 (3H, m), 5.08 (1H, m), 7.4 - 7.58 (4H, m)
IR (KBr) cm⁻¹: 1747, 1650, 1558, 1540

### Example 13

### (1R,5S,6S)-2-[(3S,5R)-5-[4-(sulfamoylaminomethyl)-phenyl]pyrrolidin-3-ylthio]-6-[(R)-1-hydroxyethyl]-1-methyl-1-carbapen-2-em-3-carboxylic acid hydrochloride

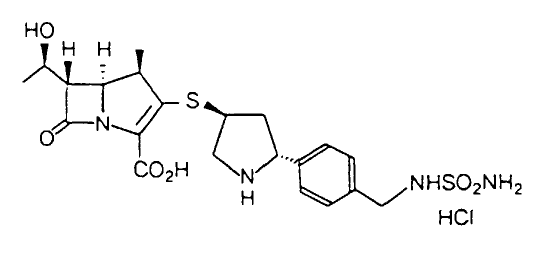

¹H-NMR (D₂O) δ: 1.24 (3H, d, J=7.0Hz), 1.29 (3H, d, J=6.5Hz), 2.12 (0.36H, m), 2.49 (0.64H, m), 2.74 (0.64H, m), 2.96 (0.36H, m), 3.36 - 3.49 (3H, m), 3.70 - 3.87 (1H, m), 4.18 - 4.28 (3H, m), 5.02 (1H, m), 7.47 - 7.50 (4H, m)
IR (KBr) cm⁻¹: 1753, 1589, 1394, 1152

### Example 14

### (1R,5S,6S)-2-[(3S,5R)-5-[4-[(1S)-aminoethyl]phenyl]-pyrrolidin-3-ylthio]-6-[(R)-1-hydroxyethyl]-1-methyl-1-carbapen-2-em-3-carboxylic acid hydrochloride

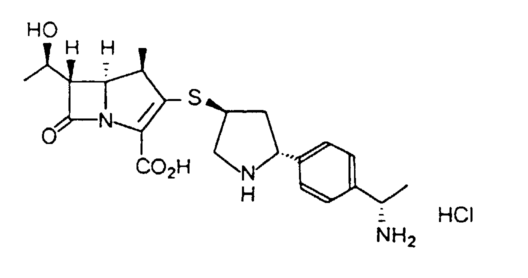

¹H-NMR (D₂O) δ: 1.22 (3H, d, J=7.3Hz), 1.27 (3H, J=6.4Hz), 1.63 (3H, d, J=6.9Hz), 2.53 (1H, dd, J=14.5, 6.9Hz), 2.79 (1H, m), 3.38 (1H, m), 3.48 (2H, m), 3.89 (1H, dd, J=12.8, 5.9Hz), 4.23 (3H, m), 4.56 (1H, q, J=6.9Hz), 5.10 (1H, dd, J=11.0, 6.8Hz), 7.54 (4H, s)
IR (KBr) cm⁻¹: 1751, 1576, 1389, 1286, 1261, 1146

### Example 15

### (1R,5S,6S)-2-[(3S,5R)-5-[4-[(1R)-aminoethyl]phenyl]-pyrrolidin-3-ylthio]-6-[(R)-1-hydroxyethyl]-1-methyl-1-carbapen-2-em-3-carboxylic acid hydrochloride

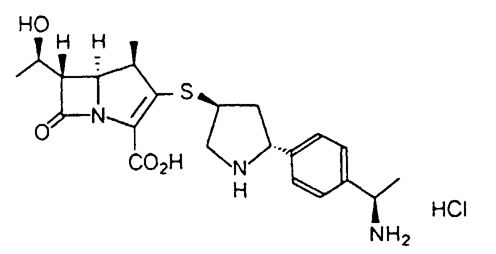

¹H-NMR (D₂O) δ: 1.22 (3H, d, J=7.3Hz), 1.26 (3H, d, J=6.4Hz), 1.62 (3H, d, J=6.9Hz), 2.52 (1H, m), 2.76 (1H, m), 3.35 (1H, m), 3.42 - 3.52 (2H, m), 3.88 (1H, m), 4.18 - 4.3 (3H, m), 4.55 (1H, m), 5.08 (1H, m), 7.48 - 7.58 (4H, m)
IR (KBr) cm⁻¹: 1747, 1650, 1558, 1540, 1394

### Example 16

### (1R,5S,6S)-2-[(3S,5R)-5-[4-(1-aminopropyl)phenyl]-pyrrolidin-3-ylthio]-6-[(R)-1-hydroxyethyl]-1-methyl-1-carbapen-2-em-3-carboxylic acid hydrochloride

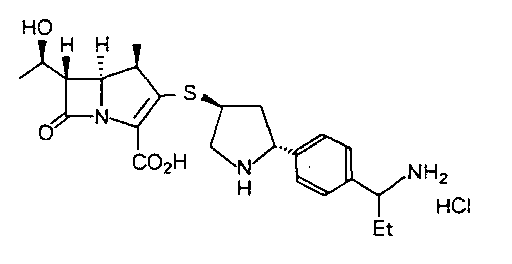

¹H-NMR (D₂O) δ: 0.83 (3H, t, d=7.4Hz), 1.21 (3H, d, J=7.3Hz), 1.26 (3H, d, J=6.3Hz), 1.99 (2H, m), 2.47 (1H, m), 2.71 (1H, m), 3.33 - 3.46 (3H, m), 3.84 (1H, dd, J=5.9, 12.6Hz), 4.18 - 4.29 (4H, m), 5.00 (1H, dd, J=6.7, 10.8Hz), 7.48 (2H, d, J=8.6Hz), 7.53 (2H, d, J=8.6Hz)
IR (KBr) cm⁻¹: 1751, 1626, 1568, 1392, 1267

### Example 17

### (1R,5S,6S)-2-[(3S,5R)-5-[4-(1,1-dimethyl-2-aminoethyl)-phenyl]pyrrolidin-3-ylthio]-6-[(R)-1-hydroxyethyl]-1-methyl-1-carbapen-2-em-3-carboxylic acid hydrochloride

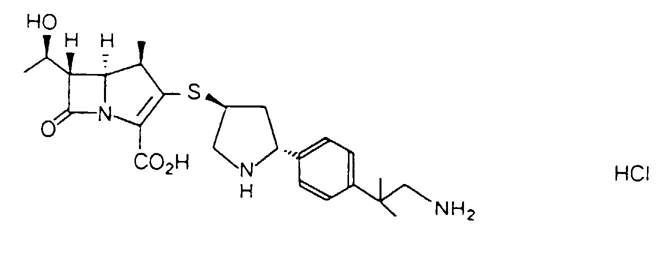

¹H-NMR (D₂O) δ: 1.22 (3H, d, J=7.1Hz), 1.27 (3H, d, J=6.5Hz), 1.42 (6H, s), 2.52 (1H, m), 2.79 (1H, m), 3.25 (2H, s), 3.37 (1H, m), 3.48 (2H, m), 3.90 (1H, m), 4.24 (3H, m), 5.08 (1H, m), 7.53 (4H, m)
IR (KBr) cm⁻¹: 1751, 1576, 1394, 1265

### Example 18

### (1R,5S,6S)-2-[(3S,5R)-5-[4-(1-amino-1-methylethyl)phenyl]pyrrolidin-3-ylthio]-6-[(R)-1-hydroxyethyl]-1-methyl-1-carbapen-2-em-3-carboxylic acid hydrochloride

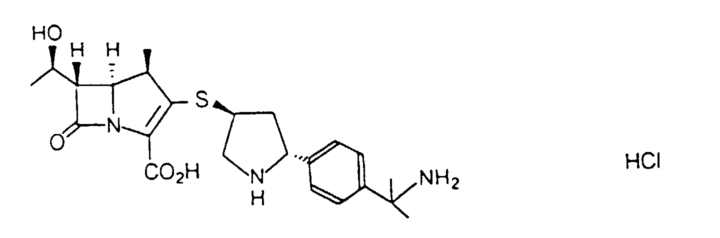

¹H-NMR (D₂O) δ: 1.23 (3H, d, J=7.3Hz), 1.27 (3H, d, J=6.5Hz), 1.74 (6H, s), 2.54 (1H, m), 2.80 (1H, m), 3.39 (1H, m), 3.50 (2H, m), 3.91 (1H, m), 4.24 (3H, m), 5.11 (1H, m), 7.58 (4H, m)
IR (KBr) cm⁻¹: 2972, 1753, 1626, 1583, 1394, 1302, 579

### Example 19

### (1R,5S,6S)-2-[(3S,5R)-5-[4-(1-amino-3-hydroxypropyl)-phenyl]pyrrolidin-3-ylthio]-6-[(R)-1-hydroxyethyl]-1-methyl-1-carbapen-2-em-3-carboxylic acid hydrochloride

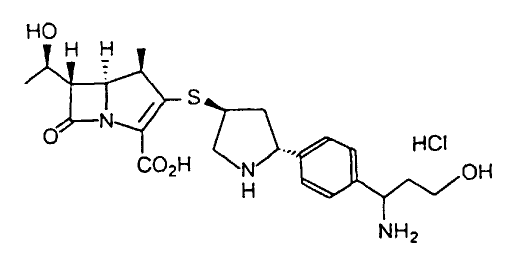

¹H-NMR (D₂O) δ: 1.23 (3H, d, J=7.3Hz), 1.27 (3H, d, J=6.5Hz), 2.08 - 2.32 (2H, m), 2.52 (1H, m), 2.73 (1H, m), 3.28 - 3.52 (4H, m), 3.60 (1H, m), 3.85 (1H, m), 4.18- 4.30 (3H, m), 4.52 (1H, m), 5.03 (1H, m), 7.51 (2H, d, J=8.6Hz), 7.55 (2H, d, J=8.6Hz)
IR (KBr) cm⁻¹: 1743, 1650, 1560, 1540

### Example 20

### (1R,5S,6S)-2-[(3S,5R)-5-[4-(1-amino-2-hydroxyethyl)phenyl]pyrrolidin-3-ylthio]-6-[(R)-1-hydroxyethyl]-1-methyl-1-carbapen-2-em-3-carboxylic acid hydrochloride

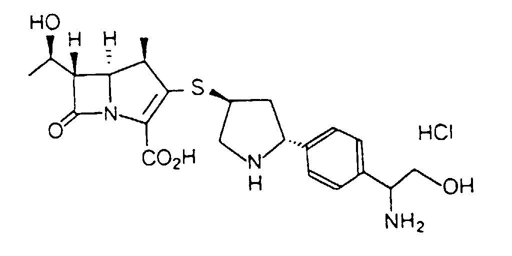

¹H-NMR (D₂O) δ: 1.23 (3H, d, J=7.3Hz), 1.27 (3H, d, J=7.0Hz), 2.54 (1H, m), 2.78 (1H, m), 3.38 (1H, m), 3.44 - 3.58 (2H, m), 3.86 - 4.02 (3H, m), 4.18 - 4.34 (3H, m), 4.51 (1H, m), 5.1 (1H, m), 7.48 - 7.62 (4H, m)
IR (KBr) cm⁻¹: 1745, 1650, 1540, 1396

### Example 21

### (1R,5S,6S)-2-[(3S,5R)-5-[4-(1-amino-2-ethylthioethyl)-phenyl]pyrrolidin-3-ylthio]-6-[(R)-1-hydroxyethyl]-1-methyl-1-carbapen-2-em-3-carboxylic acid hydrochloride

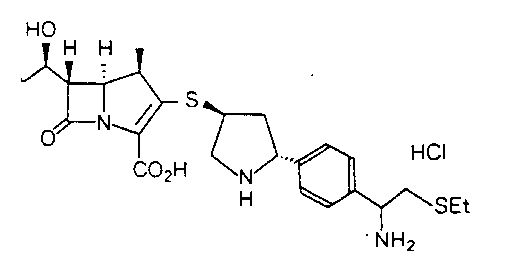

¹H-NMR (D₂O) δ: 1.17 (3H, t, J=7.4Hz), 1.23 (3H, d, J=7.2Hz), 1.28 (3H, d, J=6.3Hz), 2.51 (2H, q, J=7.3Hz), 2.55 (1H, m), 2.78 (1H, m), 3.05 - 3.22 (2H, m), 3.36 (1H, m), 3.42 - 3.52 (2H, m), 3.88 (1H, m), 4.18 - 4.30 (3H, m), 4.55 (1H, m), 5.06 (1H, m), 7.5 - 7.6 (4H, m)
IR (KBr) cm⁻¹: 1743, 1650, 1558, 1540

### Example 22

### (1R,5S,6S)-2-[(3S,5R)-5-[4-(1-N-methylamino-2-sulfamoylethyl)phenyl]pyrrolidin-3-ylthio]-6-[(R)-1-hydroxyethyl]-1-methyl-1-carbapen-2-em-3-carboxylic acid hydrochloride

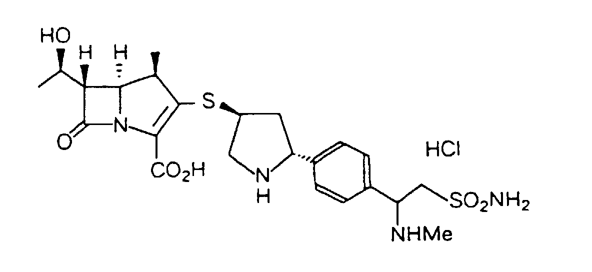

¹H-NMR (D₂O) δ: 1.22 (3H, d, J=7.0Hz), 1.27 (3H, d, J=6.3Hz), 2.25 (3H, s), 2.47 (1H, m), 2.72 (1H, m), 3.34 - 3.47 (3H, m), 3.67 - 3.87 (3H, m), 4.16 - 4.30 (4H, m), 4.99 (1H, dd, J=6.9, 10.5Hz), 7.49 (4H, s)
IR (KBr) cm⁻¹: 1751, 1587, 1394, 1319, 1151

### Example 23

### (1R,5S,6S)-2-[(3S,5R)-5-[4-(1-amino-2-thiocarbamoyl-ethyl)phenyl]pyrrolidin-3-ylthio]-6-[(R)-1-hydroxyethyl]-1-methyl-1-carbapen-2-em-3-carboxylic acid hydrochloride

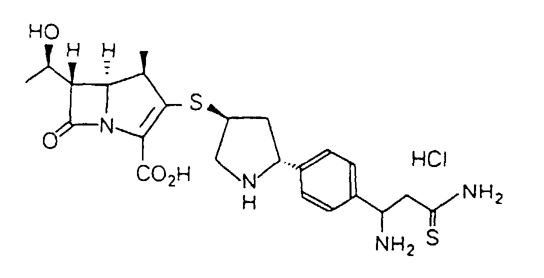

¹H-NMR (D₂O) δ: 1.23 (3H, d, J=7.3Hz), 1.28 (3H, d, J=6.4Hz), 2.55 (1H, m), 2.79 (1H, m), 3.34 (2H, m), 3.37 (1H, m), 3.48 (2H, m), 3.90 (1H, m), 4.24 (3H, m), 5.01 (1H, t, J=7.3Hz), 5.11 (1H, m), 7.56 (4H, s)
IR (KBr) cm⁻¹: 1749, 1647, 1541, 1396

### Example 24

### (1R,5S,6S)-2-[(3S,5R)-5-[4-(1-amino-2-carbamoylethyl)-phenyl]pyrrolidin-3-ylthio]-6-[(R)-1-hydroxyethyl]-1- methyl-1-carbapen-2-em-3-carboxylic acid hydrochloride

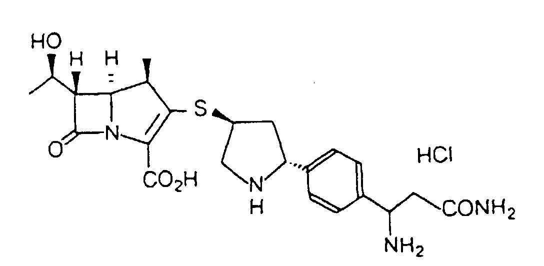

¹H-NMR (D₂O) δ: 1.21 (3H, d, J=7.3Hz), 1.26 (3H, d, J=6.3Hz), 2.3 (1H, m), 2.72 (1H, m), 2.86 - 3.08 (2H, m), 3.3 - 3.52 (3H, m), 3.84 ( 1H, m), 4.18 - 4.32 (3H, m), 5.02 (1H, m), 7.45 - 7.6 (4H, m)
IR (KBr) cm⁻¹: 1749, 1673, 1575, 1392

### Example 25

### (1R,5S,6S)-2-[(3S,5R)-5-[4-(1-aminocyclopropyl)phenyl]-pyrrolidin-3-ylthio]-6-[(R)-1-hydroxyethyl]-1-methyl-1-carbapen-2-em-3-carboxylic acid hydrochloride

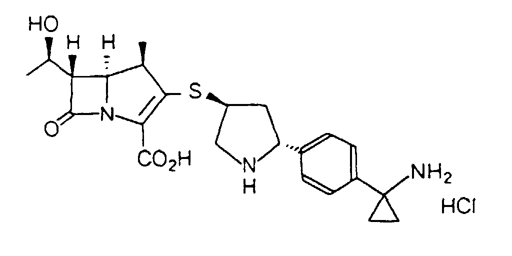

¹H-NMR (D₂O) δ: 1.16 - 1.30 (8H, m), 1.36 (2H, m), 2.49 (1H, m), 2.72 (1H, m), 3.30 - 3.50 (3H, m), 3.84 (1H, dd, J=13.0, 6.0Hz), 4.20 (3H, m), 5.03 (1H, dd, J=11.0, 7.0Hz), 7.49 (4H, s)
IR (KBr) cm⁻¹: 2966, 1749, 1558, 1349, 1261

### Example 26

### (1R,5S,6S)-2-[(3S,5R)-5-[4-(1-aminocarbamoylmethyl)phenyl]-pyrrolidin-3-ylthio]-6-[(R)-1-hydroxyethyl]-1-methyl-1-carbapen-2-em-3-carboxylic acid hydrochloride

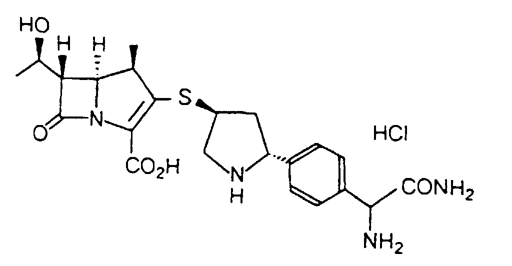

¹H-NMR (D₂O) δ: 1.22 (3H, d, J=7.3Hz), 1.26 (3H, d, J=6.3Hz), 2.53 (1H, m), 2.78 (1H, m), 3.42 - 3.57 (2H, m), 3.9 (1H, m), 4.19 - 4.30 (3H, m), 5.08 - 5.2 (2H, m), 7.52 - 7.65 (4H, m)
IR (KBr) cm⁻¹: 1749, 1697, 1394

### Example 27

### (1R,5S,6S)-2-[(3S,5R)-5-[4-(1-cyanoaminomethyl)phenyl]-pyrrolidin-3-ylthio]-6-[(R)-1-hydroxyethyl]-1-methyl-1- carbapen-2-em-3-carboxylic acid hydrochloride

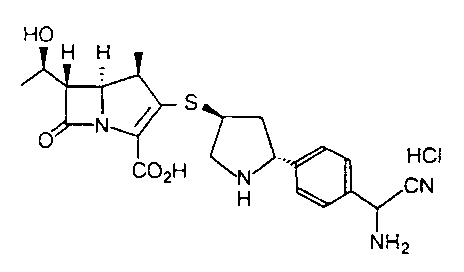

¹H-NMR (D₂O) δ: 1.23 (3H, d, J=7.3Hz), 1.27 (3H, J=6.3Hz), 2.52 (1H, m), 2.73 (1H, m), 3.3- 3.5 (3H, m), 3.78 (1H, m), 4.18 - 4.3 (2H, m), 5.02 - 5.14 (2H, m), 7.52 - 7.62 (4H, m)
IR (KBr) cm⁻¹: 1749, 1648, 1560, 1394

### Example 28

### (1R,5S,6S)-2-[(3S,5R)-5-[4-(N-acetoimidoylaminomethyl)-phenyl]pyrrolidin-3-ylthio]-6-[(R)-1-hydroxyethyl]-1-methyl-1-carbapen-2-em-3-carboxylic acid hydrochloride

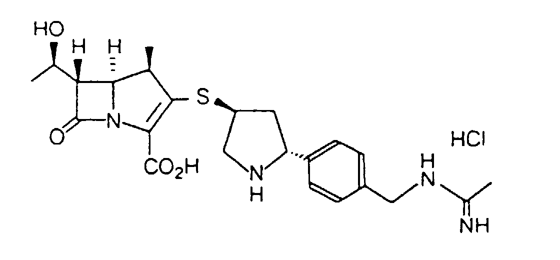

¹H-NMR (D₂O) δ: 1.21 (3H, d, J=7.1Hz), 1.26 (3H, d, J=6.3Hz), 2.26 (3H, s), 2.48 (1H, m), 2.78 (1H, m), 3.36 (1H, m), 3.4 - 3.52 (2H, m), 3.87 (1H, dd, J=12.5Hz, 5.9Hz), 4.12 - 4.26 (3H, m), 4.51 (2H, s), 5.05 (1H, m), 7.42 (2H, d, J=8.3Hz), 7.49 (2H, d, J=8.3Hz)
IR (KBr) cm⁻¹: 1749, 1681, 1648, 1560, 1349

### Example 29

### (1R,5S,6S)-2-[(3S,5R)-5-[4-(quadininomethyl)phenyl]-pyrrolidin-3-ylthio]-6-[(R)-1-hydroxyethyl]-1-methyl-1-carbapen-2-em-3-carboxylic acid hydrochloride

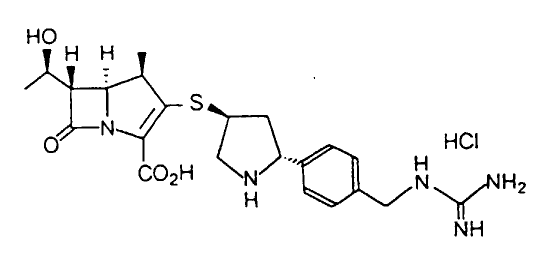

IR (KBr) cm⁻¹: 1749, 1653, 1558, 1396, 804
MS (FAB) m/z 460 (M+H)⁺

### Example 30

### (1R,5S,6S)-2-[(3S,5R)-5-(4-aminomethyl-2-naphthyl)-pyrrolidin-3-ylthio]-6-[(R)-1-hydroxyethyl]-1-methyl-1-carbapen-2-em-3-carboxylic acid hydrochloride

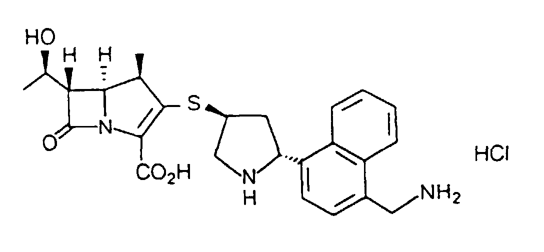

¹H-NMR (D₂O) δ: 1.23 (3H, d, J=7.0Hz), 1.26 (3H, d, J=6.3Hz), 2.61 (1H, m), 3.02 (1H, m), 3.40 (1H, m), 3.47 (1H, m), 3.53 (1H, m), 3.87 (1H, m), 4.23 (2H, m), 4.29 (1H, m), 4.70 (2H, m), 5.86 (1H, m), 7.64 (1H, m), 7.73 (3H, m), 8.16 (1H, m), 8.22 (1H, m)
IR (KBr) cm⁻¹: 1754, 1575, 721

### Example 31

### (1R,5S,6S)-2-[(3S,5R)-5-(5-aminomethylthiophen-2-yl)-pyrrolidin-3-ylthio]-6-[(R)-1-hydroxyethyl]-1-methyl-1-carbapen-2-em-3-carboxylic acid hydrochloride

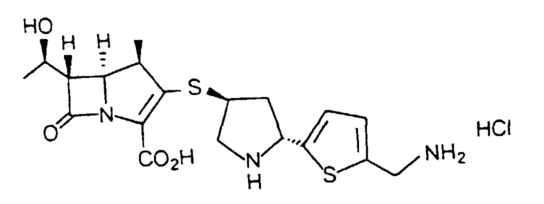

¹H-NMR (D₂O) δ: 1.21 (3H, d, J=7.2Hz), 1.25 (3H, d, J=6.3Hz), 2.51 (1H, m), 2.73 (1H, m), 3.32 (2H, m), 3.42 (1H, m), 3.74 (1H, m), 4.19 (3H, m), 4.34 (2H, s), 5.20 (1H, m), 7.16 (2H, m)
IR (KBr) cm⁻¹: 1754, 1579, 721

### Example 32

### (1R,5S,6S)-2-[(3S,5R)-5-(2-aminomethyl-4-thienyl)-pyrrolidin-3-ylthio]-6-[(R)-1-hydroxyethyl]-1-methyl-1-carbapen-2-em-3-carboxylic acid hydrochloride

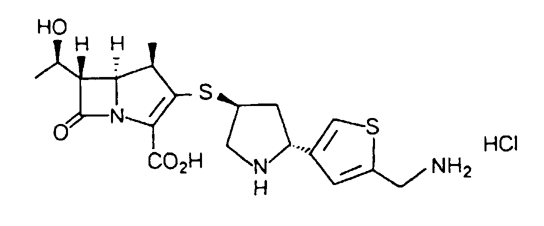

¹H-NMR (D₂O) δ: 1.19 (3H, d, J=7.3Hz), 1.24 (3H, d, J=6.3Hz), 2.44 - 2.51 (1H, m), 2.67 - 2.78 (1H, m), 3.31 - 3.42 (3H, m), 3.79 - 3.85 (1H, m), 4.16 - 4.22 (3H, m), 4.35 (2H, s), 5.03 - 5.08 (1H, m), 7.26 (1H, s), 7.61 (1H, s)
IR (KBr) cm⁻¹: 1753, 1577, 1392

### Example 33

### (1R,5S,6S)-2-[(3S,5R)-5-(2-N-methylaminomethyl-4-thienyl)pyrrolidin-3-ylthio]-6-[(R)-1-hydroxyethyl]-1-methyl-1-carbapen-2-em-3-carboxylic acid hydrochloride

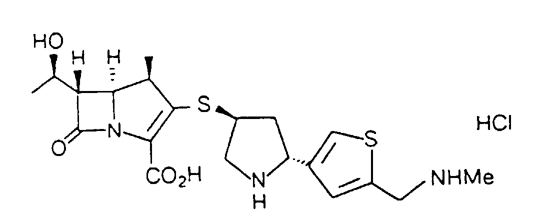

¹H-NMR (D₂O) δ: 1.20 (3H, d, J=7.3Hz), 1.25 (3H, d, J=6.5Hz), 2.45 - 2.52 (1H, m), 2.70 (3H, s), 2.67 - 2.76 (1H, m), 3.30 - 3.46 (3H, m), 3.79 - 3.85 (1H, m), 4.17 - 4.23 (3H, m), 4.40 (2H, s), 5.08 (1H, m), 7.31 (1H, s), 7.66 (1H, s)
IR (KBr) cm⁻¹: 1753, 1614, 1390

### Example 34

### (1R,5S,6S)-2-[(3S,5R)-5-(5-N-methylaminomethyl-2-thienyl)pyrrolidin-3-ylthio]-6-[(R)-1-hydroxyethyl]-1-methyl-1-carbapen-2-em-3-carboxylic acid hydrochloride

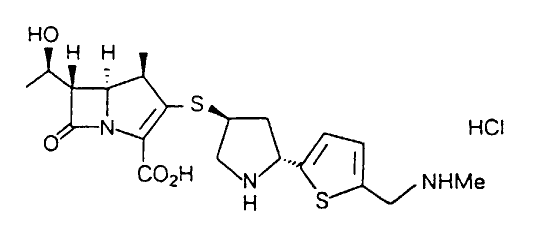

¹H-NMR (D₂O) δ: 1.17 (3H, d, J=7.2Hz), 1.25 (3H, d, J=6.5Hz), 2.27 (1H, m), 2.43 (1H, m), 2.66 (3H, s), 3.00 (1H, m), 3.36 (2H, m), 3.50 (1H, m), 3.93 (1H, m), 4.17 (2H, m), 4.34 (2H, s), 4.78 (1H, m), 7.01 (1H, d, J=3.6Hz), 7.11 (1H, d, J=3.6Hz)
IR (KBr) cm⁻¹: 1754, 1587, 1390

### Example 35

### (1R,5S,6S)-2-[(3S,5R)-5-[5-(1-aminoethyl)-2-thienyl]-pyrrolidin-3-ylthio]-6-[(R)-1-hydroxyethyl]-1-methyl-1-carbapen-2-em-3-carboxylic acid hydrochloride

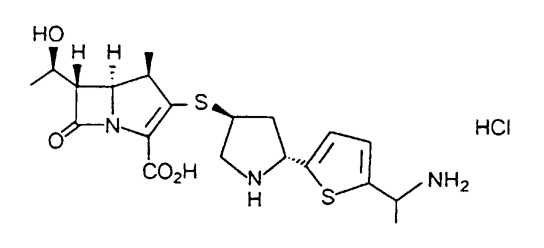

¹H-NMR (D₂O) δ: 1.20 (3H, d, J=7.0Hz), 1.26 (3H, d, J=6.4Hz), 1.68 (3H, d, J=6.9Hz), 2.47 (1H, m), 2.69 (1H, m), 3.28 - 3.45 (3H, m), 3.74 (1H, m), 4.11 - 4.23 (3H, m), 4.72 - 4.82 (1H, m), 5.15 (1H, m), 7.15 (2H, s)
IR (KBr) cm⁻¹: 1751, 1579, 1392

### Example 36

### (1R,5S,6S)-2-[(3S,5R)-5-(5-aminomethyl-2-furyl)pyrrolidin-3-ylthio]-6-[(R)-1-hydroxyethyl]-1-methyl-1-carbapen-2-em-3-carboxylic acid hydrochloride

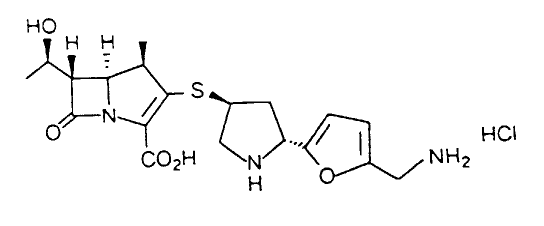

¹H-NMR (D₂O) δ: 1.21 (3H, d, J=6.9Hz), 1.26 (3H, d, J=6.3Hz), 2.40 (1H, m), 2.78 (1H, m), 3.33 - 3.46 (3H, m), 3.74 (1H, dd, J=5.7, 12.5Hz), 4.20 (2H, s), 4.16 - 4.24 (3H, m), 5.08 (1H, m), 6.54 (1H, d, J=3.3Hz), 6.59 (1H, d, J=3.3Hz)
IR (KBr) cm⁻¹: 1753, 1578, 1392, 1259

### Example 37

### (1R,5S,6S)-2-[(3S,5R)-5-[5-(N-methylaminomethyl)-2-furyl]pyrrolidin-3-ylthio]-6-[(R)-1-hydroxyethyl]-1-methyl-1-carbapen-2-em-3-carboxylic acid hydrochloride

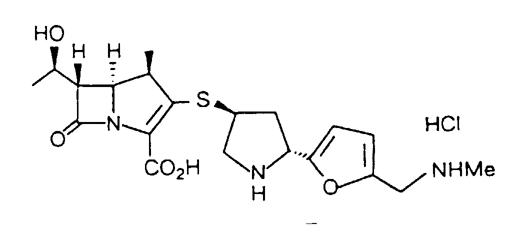

¹H-NMR (D₂O) δ: 1.21 (3H, d, J=6.9Hz), 1.26 (3H, d, J=6.3Hz), 2.40 (1H, m), 2.78 (1H, m), 3.33 - 3.46 (3H, m), 3.74 (1H, dd, J=5.7, 12.5Hz), 4.20 (2H, s), 4.16 - 4.24 (3H, m), 5.08 (1H, m), 6.54 (1H, d, J=3.3Hz), 6.59 (1H, d, J=3.3Hz)
IR (KBr) cm⁻¹: 1753, 1578, 1392, 1259

### Example 38

### (1R,5S,6S)-2-[(3S,5R)-5-(4-aminocyclohexyl)pyrrolidin-3-ylthio]-6-[(R)-1-hydroxyethyl]-1-methyl-1-carbapen-2-em-3-carboxylic acid hydrochloride

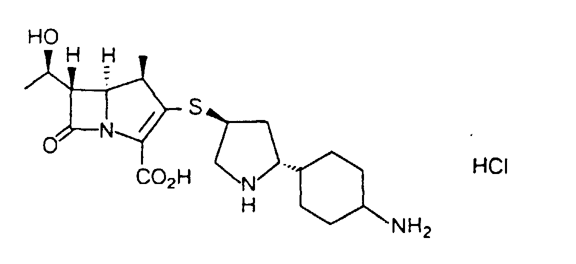

¹H-NMR (D₂O) δ: 1.32 (6H, m), 1.83 (7H, m), 2.07 (1H, m), 2.18 (1H, m), 2.32 (1H, m), 3.19 (1H, m), 3.42 (4H, m), 3.76 (2H, m), 4.17 (1H, m), 4.31 (2H, m)
IR (KBr) cm⁻¹: 1753, 1689

### Example 39

### (1R,5S,6S)-2-[(3S,5R)-5-(4-aminomethylcyclohexyl)pyrrolidin-3-ylthio]-6-[(R)-1-hydroxyethyl]-1-methyl-1-carbapen-2-em-3-carboxylic acid hydrochloride

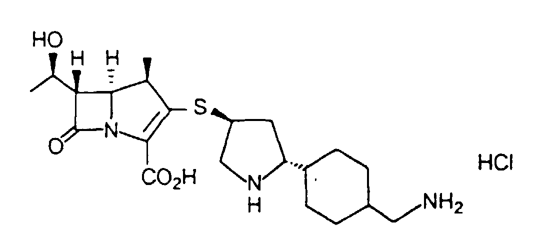

¹H-NMR (D₂O) δ: 1.04 (1H, m), 1.20 (3H, d, J=7.2Hz), 1.25 (3H, d, J=7.2Hz), 1.46 (2H, m), 1.61 (3H, m), 1.82 (3H, m), 2.22 (2H, m), 2.83 (1H, d, J=7.1Hz), 2.95 (1H, d, J=7.1Hz), 3.32 (2H, m), 3.43 (1H, m), 3.68 (2H, m), 3.89 (1H, m), 4.03 (1H, m), 4.21 (2H, m)
IR (KBr) cm⁻¹: 1749, 1581

### Example 40

### (1R,5S,6S)-2-[(3S,5R)-5-[4-[(S)-1-amino-2-(carbamoyl)ethyl]phenyl]pyrrolidin-3-ylthio]-6-[(R)-1-hydroxyethyl]-1-methyl-1-carbapen-2-em-3-carboxylic acid hydrochloride

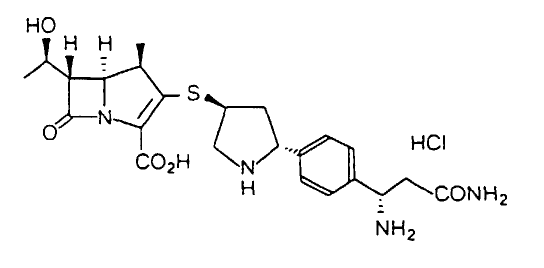

¹H-NMR (D₂O) δ: 1.04 (3H, d, J=7.0Hz), 1.08 (3H, d, J=6.4Hz), 2.36 (1H, dd, J=14.0, 7.0Hz), 2.61 (1H, m), 2.81 (1H, dd, J=15.6, 7.3Hz), 2.88 (1H, dd, J=15.6, 7.3Hz), 3.18 (1H, dq, J=8.8, 7.0Hz), 3.28 (1H, dd, J=5.8, 2.8Hz), 3.31 (1H, br d, J=12.8Hz), 3.72 (1H, dd, J=12.8, 5.8Hz), 4.05 (3H, m), 4.59 (1H, t, J=7.3Hz), 4.92 (1H, dd, J=11.0, 7.0Hz), 7.36 (4H, m)
IR (KBr) cm⁻¹: 1751, 1672, 1585, 1388, 1259, 1147, 773, 665

### Example 41

### (1R,5S,6S)-2-[(3S,5R)-5-[4-[(R)-1-amino-2-(carbamoyl)ethyl]phenyl]pyrrolidin-3-ylthio]-6-[(R)-1-hydroxyethyl]-1-methyl-1-carbapen-2-em-3-carboxylic acid hydrochloride

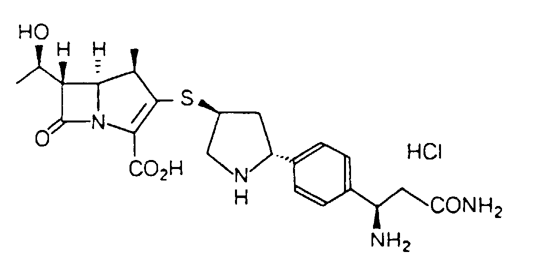

¹H-NMR (D₂O) δ: 1.04 (3H, d, J=7.3Hz), 1.08 (3H, d, J=6.4Hz), 2.36 (1H, dd, J=14.0, 6.7Hz), 2.60 (1H, m), 2.81 (1H, dd, J=15.8, 7.3Hz), 2.88 (1H, dd, J=15.8, 7.3Hz), 3.18 (1H, dq, J=9.4, 7.3Hz), 3.28 (1H, dd, J=6.1, 2.8Hz), 3.31 (1H, br d, J=12.8Hz), 3.72 (1H, dd, J=12.8, 5.8Hz), 4.05 (3H, m), 4.59 (1H, t, J=7.3Hz), 4.92 (1H, dd, J=11.0, 6.7Hz), 7.36 (4H, m)
IR (KBr) cm⁻¹: 3417, 1751, 1673, 1583, 1390, 1261, 1147, 572

### Formulation Example 1

| | |
|---|---|
| The compound of Example 40 | 10 (parts) |
| Heavy magnesium oxide | 15 |
| Lactose | 75 |

The ingredients mentioned above were uniformly mixed together to prepare a powdery or fine-powdery composition having a size of 350 µm or less. The powder thus obtained was introduced into a capsule container to obtain a capsule agent.

### Formulation Example 2

| | |
|---|---|
| The compound of Example 41 | 10 (parts) |
| Starch | 15 |
| Lactose | 16 |
| Crystalline cellulose | 21 |
| Polyvinyl alcohol | 3 |
| Distilled water | 30 |

The ingredients mentioned above were uniformly mixed together, ground, granulated, dried, and then classified by means of sieves to obtain a granular agent having a size of from 1,410 to 177 µm.

### Formulation Example 3

A granular agent was prepared by the same method as in Formulation Example 2. 4 parts of calcium stearate was added to 96 parts of the granular agent and the mixture was compression-molded to obtain tablets having a diameter of 10 mm.

### Formulation Example 4

To 90 parts of a granular agent obtained by the same method as in Formulation Example 2 were added 10 parts of crystalline cellulose and 3 parts of calcium stearate, and the mixture was compression-molded to obtain tablets having a diameter of 8 mm. By adding thereto a mixed suspension of syrup gelatin and precipitated calcium carbonate, there were obtained sugar-coated tablets.

### Formulation Example 5

| | |
|---|---|
| The compound of Example 1 | 10 (parts) |
| Non-ionic surfactant | 2.4 |
| Physiological salt solution | 97 |

The ingredients mentioned above were mixed together at a lukewarm temperature and the resulting solution was introduced into an ample and sterilized to obtain an injection

### Referential Example 1

### (2R,4S)-4-Acetylthio-1-allyloxycarbonyl-2-(4-N-allyloxycarbonyl-N-methylaminomethyl)phenylpyrrolidine

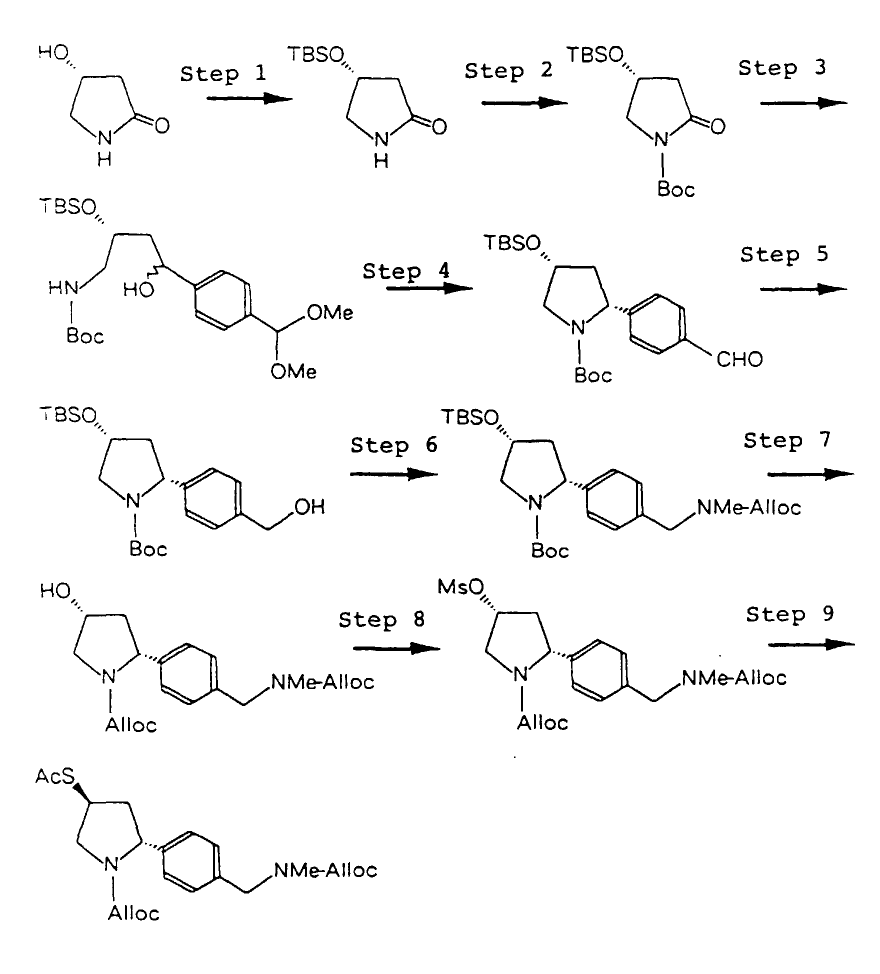

### (Step 1)

Under a stream of nitrogen, t-butyldimethylsilyl chloride (784 g, 5.2 mol) and imidazole (506 g, 7.43 mol) were successively added at an ice-cooled temperature to a solution of 4-(R)-hydroxy-2-pyrrolidone (500 g, 4.95 mol) in N,N,-dimethylformamide (hereinafter, referred to as DMF) (2,500 ml). The reaction mixture was stirred at the same temperature as above for 30 minutes, and then the mixture was poured into water (3,000 ml). The resulting precipitate was collected by filtration with suction, and washed with water. The precipitate was dissolved in chloroform, the organic layer was dried on anhydrous sodium sulfate, and the solution was evaporated under reduced pressure to obtain 4-t-butyldimethylsiloxy-2-pyrrolidone (1,054 g, yield 99.0%) as a white solid.
¹H-NMR (CDCl₃) δ: 0.08 (6H, s), 0.89 (9H, s), 2.27 (1H, dd, J=17.0, 4.3Hz), 2.25 (1H, dd, J=17.0, 6.8Hz), 3.23 (1H, dd, J=9.9, 3.5Hz), 3.58 (1H, dd, J=9.9, 6.1Hz), 4.56 (1H, dddd, J=6.8, 6.1, 4.3, 3.5Hz), 5.66 (1H, br-s)

### (Step 2)

Under a stream of nitrogen, a solution of di-t-butyl dicarbonate (1,123 g, 5.14 mol) in acetonitrile (1,000 ml) was added to a mixed solution of the compound obtained in Step 1 (1,054 g, 4.9 mol), N,N-dimethylaminopyridine (340 g, 2.78 mol) and triethylamine (298 g, 2.94 mol) in acetonitrile (5,000 ml) at an ice-cooled temperature over a period of one hour. After stirring the reaction mixture at ambient temperature for 12 hours, ethyl acetate was added to the reaction mixture, the organic layer was successively washed with water, 1N HCl, 1N NaOH and brine and dried on anhydrous sodium sulfate, and the solution was evaporated under reduced pressure. The solid product thus formed was washed with hexane and dried to obtain 1-t-butoxycarbonyl-4-t-butyldimethylsiloxy-2-pyrrolidone (1,423 g, yield 92.1%) as a white solid product.
¹H-NMR (CDCl₃) δ: 0.08 (6H, s), 0.88 (9H, s), 1.53 (9H, s), 2.45 (1H, dd, J=17.3, 3.4Hz), 2.72 (1H, dd, J=17.4, 6.3Hz), 3.62 (1H, dd, J=11.4, 3.0Hz), 3.86 (1H, dd, J=11.2, 5.3Hz), 4.56 (1H, dddd, J=6.3, 5.3, 3.4, 3.0Hz)

### (Step 3)

Under a stream of nitrogen, 4-bromobenzaldehyde dimethyl acetal (806.6 g, 3.49 mol) was added at ambient temperature to a solution of magnesium (170 g, 6.98 mol) in THF (3,000 ml) over a period of one hour. After stirring the reaction mixture for additional 30 minutes, a solution of the compound obtained in Step 2 (550 g, 1.74 mol) in THF (4,000 ml) was added thereto at an ice-cooled temperature over a period of 90 minutes. Under a stream of nitrogen, at an ice-cooled temperature, sodium borohydride (99.1 g, 2.61 mol) and methanol (3,500 ml) were successively added to the reaction mixture. The mixture was poured into a mixture of ethyl acetate and brine, the organic layer was washed successively with water and brine and dried on anhydrous sodium sulfate, and the solution was evaporated under reduced pressure. The residue thus obtained was purified by silica gel column chromatography (Wakogel® C-300, hexane-ethyl acetate 5:1 to 4:1) to obtain (3R)-4-t-butoxycarbonylamino-3-t-butyldimethylsiloxy-1-(4-dimethoxymethylphenyl)butanol (458.6 g, yield 56.0%) as a colorless oil.
¹H-NMR (CDCl₃) δ: 0.11 (6H, s), 0.92 (9H, s), 1.42 (9H, s), 1.86 (2H, m), 3.20 (1H, m), 3.31 (6H, s), 4.08 (1H, m), 4.77 (1H, m), 4.90 (1H, m), 5.37 (1H, s), 7.33 (2H, d, J=7.0Hz), 7.41 (2H, d, J=7.0Hz)

### (Step 4)

Under a stream of nitrogen, triethylamine (1,703 g, 16.83 mol) and methanesulfonyl chloride (707g, 6.17 mol) were successively added to a solution of the compound obtained in Step 3 (2,631.6 g, 5.61 mol) in methylene chloride (53 l) at -60°C. The reaction mixture was poured into water, the organic layer was washed successively with water and brine and dried on anhydrous sodium sulfate, and the solution was evaporated under reduced pressure. After dissolving the residue thus obtained in a mixture of water (1.5 l) and THF (10 l), p-toluenesulfonic acid monohydrate (106 g, 561 mmol) was added thereto, and the mixture was stirred at ambient temperature for one hour. The reaction mixture was poured into ethyl acetate, the organic layer was washed successively with water and brine and dried on anhydrous sodium sulfate, and the solution was evaporated under reduced pressure. The residue thus obtained was dissolved in hexane and the resulting precipitate was collected by filtration with suction to obtain (2R,4R)-1-t-butoxycarbonyl-4-t-butyldimethylsiloxy-2-(4-formylphenyl)pyrrolidine (862.6 g, yield 38.0%) as a white solid product.
¹H-NMR (CDCl₃) δ: 0.03 (6H, s), 0.72 (9H, s), 1.18 (6H, s), 1.43 (3H, s), 1.88 (1H, m), 2.48 (1H, m), 3.43 (1H, m), 3.80 (1H, m), 4.40 (1H, m), 4.81 (0.66H, m), 4.79 (0.34H, m), 7.40 (2H, d, J=7.0Hz), 7.78 (2H, d, J=7.0Hz), 9.96 (1H, s)

### (Step 5)

Under a stream of nitrogen, sodium borohydride (14.8 g, 390 mol) was added to a solution of the compound obtained in Step 4 (143.9 g, 355 mmol) in methanol (3,000 ml) at an ice-cooled temperature, and stirred at the same temperature as above for 30 minutes. The reaction mixture was poured into water, the organic layer was washed successively with water and brine and dried on anhydrous sodium sulfate, and the solution was evaporated under reduced pressure. The residue thus obtained was purified by silica gel column chromatography (Wakogel® C-300, hexane-ethyl acetate 5:1 to 4:1) to obtain (2R,4R)-1-t-butoxycarbonyl-4-t-butyldimethylsiloxy-2-(4-hydroxymethylphenyl)pyrrolidine (130.1 g, yield 90.0%) as a colorless oil.
¹H-NMR (CDCl₃) δ: 0.07 (6H, s), 0.78 (9H, s), 1.16 (6H, s), 1.41 (3H, s), 1.82 (1H, m), 2.45 (1H, m), 3.39 (1H, m), 3.84 (1H, m), 4.33 (1H, m), 4.62 (2H, s), 4.70 (0.66H, m), 4.83H (0.34H, m), 7.23 (4H, s)

### (Step 6)

Under a stream of nitrogen, triethylamine (64.8 g, 640 mol) and methanesulfonyl chloride (27.2 ml, 352 mmol) were successively added to a solution of the compound obtained in Step 5 (130.1 g, 320 mmol) in methylene chloride (2,600 ml) at -30°C. The reaction mixture was poured into water, the organic layer was washed successively with water and brine and dried on anhydrous sodium sulfate, and the solution was evaporated under reduced pressure. The residue thus obtained was dissolved in methylene chloride, a 40% methanolic solution of methylamine (1,488 ml, 19.2 mol) was added thereto at -10°C, and the resulting mixture was stirred at the same temperature as above for 15 minutes. After distilling off the solvent under reduced pressure, the residue thus obtained was dissolved in a mixture of 1,4-dioxane (3,000 ml) and water (500 ml), to which was added allyloxycarbonyl chloride (46.3 g, 384 mmol) at 5°C while keeping pH 9.0 with 5N NaOH. The reaction mixture was poured into ethyl acetate, the organic layer was washed successively with water and brine and dried on anhydrous sodium sulfate, and the solution was evaporated under reduced pressure. The residue thus obtained was purified by silica gel column chromatography (Wakogel® C-300, hexane-ethyl acetate 5:1) to obtain (2R,4R)-1-t-butoxycarbonyl-4-t-butyldimethylsiloxy-2-[4-(N-allyloxycarbonyl-N- methylaminomethyl)phenyl]pyrrolidine (110.5 g, yield 68.5%) as a colorless oil.
¹H-NMR (CDCl₃) δ: 0.02 (3H, s), 0.06 (3H, s), 0.78 (9H, s), 1.16 (6H, s), 1.42 (3H, s), 1.87 (1H, m), 2.49 (1H, m), 2.82 (1.4H, s), 2.83 (1.6H, s), 3.40 (1H, m), 3.83 (1H, m), 4.36 (1H, m), 4.42 (2H, m), 4.62 (2H, m), 4.70 (0.66H, m), 4.89 (0.34H, m), 5.27 (1H, m), 5.95 (1H, m), 7.15 (2H, d, J=8.2Hz), 7.20 (2H, d, J=8.2Hz)

### (Step 7)

Concentrated hydrochloric acid (83 ml, 1 mol) was added to a solution of the compound obtained in Step 6 (110.5 g, 219 mmol) in methanol (1,200 ml), and heated under reflux for 30 minutes. After distilling off the solvent, the residue was dissolved in a mixture of 1,4-dioxane (1,000 ml) and water (200 ml), to which was added allyloxycarbonyl chloride (46.3 g, 384 mmol) at 5°C while keeping pH 9.0 with 5N NaOH. The reaction mixture was poured into ethyl acetate, the organic layer was washed successively with water and brine and dried on anhydrous sodium sulfate, and then the solution was evaporated under reduced pressure. The residue thus obtained was purified by silica gel column chromatography (Wakogel® C-300, hexane-ethyl acetate 1:2) to obtain (2R,4R)-1-allyloxycarbonyl-4-hydroxy-2-[4-(N-allyloxycarbonyl-N-methylaminomethyl)-phenyl]pyrrolidine (82.0 g, yield 100%) as a colorless oil.
¹H-NMR (CDCl₃) δ: 2.01 (1H, m), 2.59 (1H, m), 2.84 (3H, s), 3.62 (1H, dd, J=11.8, 3.6Hz), 3.91 (1H, m), 4.38 - 4.58 (5H, m), 4.62 (2H, d, J=5.5Hz), 4.83 - 5.04 (2H, m), 5.27 (3H, m), 5.54 - 6.02 (2H, m), 7.20 (2H, d, J=9.3Hz), 7.25 (2H, d, J=9.3Hz)

### (Step 8)

Under a stream of nitrogen, triethylamine (61.05 ml, 438 mol) and methanesulfonyl chloride (18.65 ml, 241 mol) were successively added to a solution of the compound obtained in Step 7 (81.99 g, 219 mmol) in methylene chloride (1,600 ml) at -40°C. The reaction mixture was poured into water, the organic layer was washed successively with saturated aqueous solution of sodium hydrogen carbonate and brine and dried on anhydrous sodium sulfate, and the solution was evaporated under reduced pressure. The residue thus obtained was purified by silica gel column chromatography (Wakogel® C-300, hexane-ethyl acetate 3:2) to obtain (2R,4R)-1-allyloxycarbonyl-4-methanesulfonyloxy-2-[4-(N-allyloxycarbonyl-N-methylaminomethyl)phenyl]pyrrolidine (91.16 g, yield 92.0%) as a colorless oil.
¹H-NMR (CDCl₃) δ: 2.22 (1H, m), 2.63 (1H, m), 2.80 (3H, s), 2.91 (3H, s), 3.71 (1H, m), 3.86 (1H, m), 4.41 - 4.62 (5H, m), 4.61 (2H, d, J=5.8Hz), 4.85 - 5.06 (2H, m), 5.29 (3H, m), 5.54 - 6.06 (2H, m), 7.20 (2H, d, J=8.8Hz), 7.24 (2H, d, J=8.8Hz)

### (Step 9)

Under a stream of nitrogen, potassium thioacetate (46.1 g, 403 mmol) was added to a solution of the compound obtained in Step 8 (91.15 g, 201 mmol) in DMF (3,000 ml) at ambient temperature, and the resulting mixture was stirred at 60°C for 3 hours. The reaction mixture was poured into a mixture of ethyl acetate and water, the organic layer was washed successively with water and brine and dried on anhydrous sodium sulfate, and the solution was evaporated under reduced pressure. The residue thus obtained was purified by silica gel column chromatography (Wakogel® C-300, hexane-ethyl acetate 2:1) to obtain the title compound (83.93 g, yield 96.3%) as a colorless oil.
¹H-NMR (CDCl₃) δ: 2.26 (1H, m), 2.32 (3H, s), 3.36 (1H, m), 2.86 (1.5H, s), 2.87 (1.5H, s), 3.62 (1H, m), 4.09 (2H, m), 4.46 (2H, s), 4.56 (1H, m), 4.64 (2H, d, J=5.5Hz), 4.82 - 5.08 (2H, m), 5.24 - 5.38 (4H, m), 5.53-6.04 (2H, m), 7.21 (4H, s)

### Referential Example 2

### (4S)-acetylthio-1-allyloxycarbonyl-2-[4-(N-allyloxycarbonyl-N-methylaminomethyl)phenyl]pyrrolidine

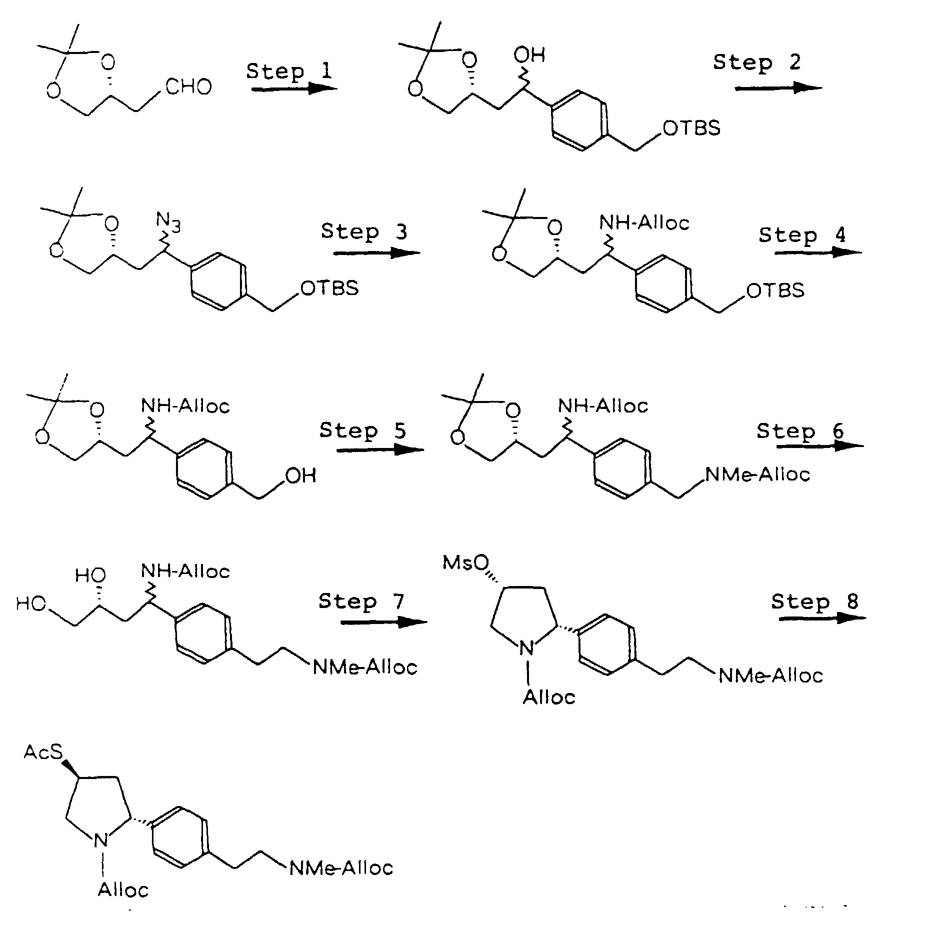

The starting compound used in Step 1, i.e. (3R)-3,4-O-isopropylidene-3,4-dihydroxybutanal, can be produced according to the method of Mori et al. [K. Mori, T. Tanigawa, T. Matsuo, Tetrahedron, 35, 436 (1979)].

### (Step 1)

Under a stream of nitrogen, 1.6M solution of n-butyllithium in hexane (27.9 ml, 44.7 mmol) was added at -78°C to a solution of 4-(t-butyldimethylsiloxymethyl)bromobenzene (14.67 g, 48.7 mmol) in THF (300 ml), and the reaction mixture was stirred at the same temperature as above for 20 minutes. Under a stream of nitrogen, a solution of (3R)-3,4-O-isopropylidene-3,4-dihydroxybutanal (5.86 g, 40.6 mmol) in THF (25 ml) was added to the reaction mixture at - 78°C. The resulting mixture was poured into a mixture of ethyl acetate and saturated aqueous solution of ammonium chloride. The organic layer was washed successively with water and brine and dried on anhydrous sodium sulfate, and the solution was evaporated under reduced pressure. The residue thus obtained was purified by silica gel column chromatography (Wakogel® C-300, hexane-ethyl acetate 7:3) to obtain (3R)-1-(4-t-butyldimethylsiloxyphenyl)-3,4-O-isopropylidene-3,4-dihydroxybutanol (9.54 g, yield 63.9 g) as a colorless oil.
¹H-NMR (CDCl₃) δ: 0.09 (6H, s), 0.92 (9H, s), 1.37 (3H, s), 1.46 (3H, s), 1.94 (2H, m), 3.57 (1H, m), 4.05 (1H, m), 4.26 (1H, m), 4.73 (2H, s), 4.92 (1H, m), 7.27 (2H, d, J=9.3Hz), 7.34 (2H, d, J=9.3Hz)

### (Step 2)

Under a stream of nitrogen, triphenylhosphine (9.84 g, 37.5 mmol), diphenylphosphoryl azide (7.7 ml, 35.7 mmol) and diethyl azodicarboxylate (5.62 ml, 35.7 mmol) were successively added to a solution of the compound obtained in Step 1 (6.57 g, 17.87 mmol) in THF (150 ml) at an ice-cooled temperature, and the reaction mixture was stirred at the same temperature as above for 20 minutes. After distilling off the solvent under reduced pressure, the residue thus obtained was purified by silica gel column chromatography (Wakogel® C-300, hexane-ethyl acetate 20:1) to obtain (3R)-1-(4-t-butyldimethylsiloxymethylphenyl)-3,4-O-isopropylidene-3,4-dihydroxybutyl azide (5.14 g, yield 73.4%) as a colorless oil.
¹H-NMR (CDCl₃) δ: 0.10 (6H, s), 0.93 (9H, s), 1.30 (1H, s), 1.38 (2H, s), 1.41 (3H, s), 1.92 (2H, m), 3.52 (1H, m), 4.09 (1H, m), 4.29 (1H, m), 4.69 (1H, m), 4.74 (2H, s), 7.30 (4H, m)

### (Step 3)

Triphenylphosphine (6.9 g, 26.3 mmol) and water (1.4 ml) were successively added to a solution of the compound obtained in Step 2 (5.14 g, 13.13 mmol) in THF (120 ml) at ambient temperature, and the reaction mixture was stirred at 50°C for 5 hours. After distilling off the solvent under reduced pressure, the residue was dissolved in a mixture of 1,4-dioxane (50 ml) and water (25 ml), to which was added at 5°C allyloxycarbonyl chloride (2.1 ml, 19.7 mmol) while keeping pH 9.0 with 5N NaOH. The reaction mixture was poured into ethyl acetate, the organic layer was washed successively with water and brine and dried on anhydrous sodium sulfate, and then the solution was evaporated under reduced pressure. The residue thus obtained was purified by silica gel column chromatography (Wakogel® C-300, hexane-ethyl acetate 4:1) to obtain (3R)-N-allyloxycarbonyl-1-(4-t-butyldimethylsiloxymethyl-phenyl)-3,4-O-isopropylidene-3,4-dihydroxybutylamine (5.46 g, yield 83.2%).
¹H-NMR (CDCl₃) δ: 0.10 (6H, s), 0.93 (9H, s), 1.31 (3H, s), 1.42 (1.5H, s), 1.44 (1.5H, s), 1.92 (1H, m), 2.08 (1H, m), 3.51 (1H, m), 3.94 (1H, m), 4.52 (2H, m), 4.71 (2H, s), 4.96 (1H, m), 5.25 (2H, m), 5.84 (1H, m), 7.24 (2H, d, J=7.6Hz), 7.29 (2H, d, J=7.6Hz)

### (Step 4)

Under a stream of nitrogen, tetra-n-butylammonium fluoride (7.5 ml, 7.5 mmol) was added to a solution of the compound obtained in Step 3 (2.5 g, 5 mmol) in THF (50 ml) at an ice-cooled temperature, and the reaction mixture was stirred at the same temperature as above for one hour. The reaction mixture was poured into a mixture of ethyl acetate and saturated solution of ammonium chloride, the organic layer was washed successively with water and brine and dried on anhydrous sodium sulfate, and then the solution was evaporated under reduced pressure. The residue thus obtained was purified by silica gel column chromatography (Wakogel® C-300, hexane-ethyl acetate 2:1 to 1:1) to obtain (3R)-N-allyloxycarbonyl-1-(4-hydroxymethylphenyl)-3,4-O-isopropylidene-3,4-dihydroxybutylamine (1.678 g, yield 100%) as a colorless oil.
¹H-NMR (CDCl₃) δ: 1.31 (3H, s), 1.42 (1H, s), 1.44 (2H, s), 1.92 (1H, m), 2.08 (1H, m), 3.50 (1H, m), 3.98 (2H, m), 4.53 (2H, m), 4.67 (2H, s), 4.96 (1H, m), 5.27 (2H, m), 5.90 (1H, m), 7.30 (4H, m)

### (Step 5)

Under a stream of nitrogen, triethylamine (3.48 ml, 25.0 mmol) and methanesulfonyl chloride (580 ml, 7.5 mmol) were successively added to a solution of the compound obtained in Step 4 (1.678 g, 5.0 mmol) in methylene chloride (50 ml) at an ice-cooled temperature, and the reaction temperature was stirred at the same temperature as above for 20 minutes. Then, 40% methanolic solution of methylamine (30 ml) was added to the reaction mixture, and stirred at an ice-cooled temperature for 20 minutes. After distilling off the solvent under reduced pressure, the residue was dissolved in a mixture of 1,4-dioxane (40 ml) and water (20 ml), and allyloxycarbonyl chloride (0.8 ml, 7.54 mmol) was added thereto at 10°C while keeping pH 9.0 with 1N NaOH. The reaction mixture was poured into ethyl acetate, the organic layer was washed successively with saturated aqueous solution of sodium hydrogen carbonate and brine and dried on anhydrous sodium sulfate, and the solution was evaporated under reduced pressure. The residue thus obtained was purified by silica gel column chromatography (Wakogel® C-300, hexane-ethyl acetate, 2:1 to 1:1) to obtain (3R)-N-allyloxycarbonyl-1-[4-(N-allyloxycarbonyl-N-methylaminomethyl)phenyl]-3,4-O- isopropylidene-3,4-dihydroxybutylamine (1.975 g, yield 91.3%) as a colorless oil.
¹H-NMR (CDCl₃) δ: 1.32 (3H, s), 1.42 (1H, s), 1.44 (2H, s), 1.91 (1H, m), 2.08 (1H, m), 2.87 (3H, s), 3.50 (1H, m), 3.96 (2H, m), 4.46 (2H, s), 4.52 (2H, m), 4.63 (2H, d, J=5.4Hz), 4.98 (1H, m), 5.25 (4H, m), 5.90 (2H, m), 7.26 (4H, m)

### (Step 6)

To a solution of the compound obtained in Step 5 (1.975 g, 4.566 mmol) in methanol (40 ml) was added 1N HCl (10 ml, 10 mmol) at ambient temperature. The reaction mixture was stirred at the same temperature as above for 4 hours. After distilling off the solvent under reduced pressure, the residue was poured into a mixture of ethyl acetate and water, the organic layer was washed successively with saturated aqueous solution of sodium hydrogen carbonate and brine and dried on anhydrous sodium sulfate, and the solution was evaporated under reduced pressure. The residue thus obtained was purified by silica gel column chromatography (Wakogel® C-300, hexane-ethyl acetate 1:2 to 1:4) to obtain (3R)-N-allyloxycarbonyl-1-[4-(N-allyloxycarbonyl-N-methylaminomethyl)phenyl]-3,4-dihydroxybutylamine (1.792 g, yield 100%) as a colorless oil.
¹H-NMR (CDCl₃) δ: 1.81 (1H, m), 2.18 (1H, m), 2.88 (3H, s), 3.46 (1H, m), 3.61 (1H, m), 3.80 (1H, m), 4.45 (2H, s), 4.56 (2H, m), 4.62 (2H, m), 4.98 (1H, m), 5.30 (4H, m), 5.92 (2H, m), 7.23 (4H, m)

### (Step 7)

Under a stream of nitrogen, triethylamine (3.4 ml, 24.4 mmol) and methanesulfonyl chloride (944 ml, 12.2 mmol) were successively added to a solution of the compound obtained in Step 6 (1.596 g, 4.067 mmol) in methylene chloride (80 ml) at an ice-cooled temperature, and the reaction mixture was stirred at the same temperature as above for 20 minutes. After distilling off the solvent under reduced pressure, the residue was poured into a mixture of ethyl acetate and water, the organic layer was washed successively with saturated aqueous solution of sodium hydrogen carbonate and brine and dried on anhydrous sodium sulfate, and the solution was evaporated under reduced pressure. Under a stream of nitrogen, the residue thus obtained was dissolved in THF (100 ml), and potassium t-butoxide (502 mg, 4.47 mmol) was added thereto at -50°C. Temperature of the reaction mixture was gradually elevated to -20°C. Then, the reaction mixture was poured into a mixture of ethyl acetate and saturated aqueous solution of ammonium chloride, the organic layer was washed successively with saturated aqueous solution of sodium hydrogen carbonate and brine and dried on anhydrous sodium sulfate, and then the solution was evaporated under reduced pressure. The residue thus obtained was purified by silica gel column chromatography (Wakogel® C-300, hexane-ethyl acetate 1:1 to 1:2) to obtain (4R)-1-allyloxycarbonyl-4-methanesulfonyloxy-2-[4-(N-allyloxycarbonyl-N- methylaminomethyl)phenyl]pyrrolidine (1.81 g, yield 98.4%) as a colorless oil.
¹H-NMR (CDCl₃) δ: 2.37 (0.3H, m), 2.51 (0.7H, m), 2.67 (0.3H, m), 2.70 (0.7H, m), 2.85 (3H, s), 2.96 (1.3H, s), 3.07 (1.7H), 3.90 (1H, m), 4.10 (1H, m), 4.46 (2H, m), 4.55 (2H, m), 4.62 (2H, m), 4.72 - 5.12 (2H, m), 5.24 (4H, m), 5.92 (2H, m), 7.19 (4H, m)

### (Step 8)

Under a stream of nitrogen, potassium thioacetate (1.37 g, 12.0 mmol) was added to a solution of the compound obtained in Step 7 (1.81 g, 4.0 mmol) in DMF (80 ml) at ambient temperature. The reaction mixture was stirred at 60°C for 3 hours. The reaction mixture was poured into a mixture of ethyl acetate and water, the organic layer was washed successively with water and brine and dried on anhydrous sodium sulfate, and then the solution was evaporated under reduced pressure. The residue thus obtained was purified by silica gel column chromatography (Wakogel® C-300, hexane-ethyl acetate 2:1) to obtain the title compound (1.626 g, yield 94.0%) as a colorless oil.
¹H-NMR (CDCl₃) δ: 1.88 (1H, m), 2.27 (1H, m), 2.30 (1H, s), 2.31 (2H, s), 2.86 (3H, s), 3.60 (1H, m), 4.04 (1H, m), 4.43 (2H, s), 4.53 (2H, m), 4.62 (2H, m), 4.82 - 5.08 (2H, m), 5.16 - 5.37 (4H, m), 5.91 (2H, m), 7.18 (4H, m)

### INDUSTRIAL UTILIZABILITY

The compound of this invention is a novel compound not found in literature and exhibits a broad antibacterial spectrum and an intense antibacterial activity against gram-positive bacteria and gram-negative bacteria and has an excellent stability to β-lactamase. Accordingly, the compound of this invention is useful as an antibacterial agent. Further, the compound of this invention further has a metallo-β-lactamase inhibitory activity, and therefore is expected to make a great contribution to the therapy of difficult-to-treat infectious diseases, etc.

## Claims

1. A compound represented by the general formula [I]: 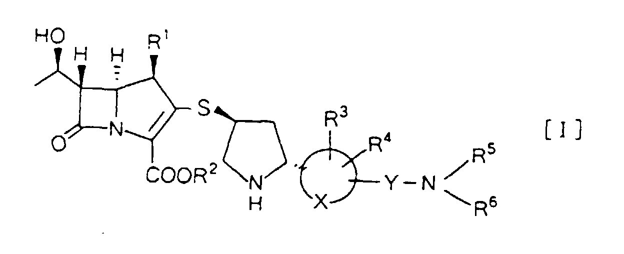 wherein R¹ represents a hydrogen atom or a lower alkyl group; R² represents a hydrogen atom, an ester residue or an alkali metal; R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group; R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxy group;
the group represented by the formula 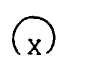 represents an aryl group selected from the group consisting of a benzene ring and a naphthalene ring, a heteroaromatic group selected from the group consisting of a pyrrole group, a furan ring and a thiophene ring, or a 5- to 8-membered cycloalkyl group; Y represents a single bond, or a 3- to 6-membered cycloalkylene group or a straight or branched chain alkylene group having 1 to 4 carbon atoms which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group; provided that a case where R³ and R⁴ both represent a hydrogen atom, the group represented by the formula 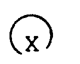 is a benzene ring, Y is a methylene group and R⁵ and R⁶ both represent a hydrogen atom is excepted.

2. A compound according to Claim 1, wherein R³ and R⁴, identical or different, represent a hydrogen atom, a halogen atom, a lower alkyl group, a carbamoyl group or a hydroxy group.

3. A compound according to Claim 1 or 2, wherein R³ and R⁴, identical or different, represent a hydrogen atom, a halogen atom or a lower alkyl group.

4. A compound according to Claim 1, wherein R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group and a hydroxy group.

5. A compound according to Claim 1 or 4, wherein R⁵ and R⁶, identical or different, represent a hydrogen atom, a lower alkoxy group, an amino-substituted lower alkyl carbonyl group, a sulfamoyl group, a lower alkylimidoyl group, an amidino group or a lower alkyl group which may have a substituent selected from the group consisting of a carbamoyl group and a hydroxy group.

6. A compound according to Claim 1, wherein said group represented by the formula 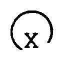 represents an aryl group selected from the group consisting of a benzene ring and a naphthalene ring, a heteroaromatic group selected from the group consisting of a furan ring and a thiophene ring, or a 5- or 6-membered cycloalkyl group.

7. A compound according to Claim 1 or 6, wherein the group represented by the formula 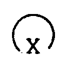 represents an aryl group selected from the group consisting of a benzene ring and a naphthalene ring, a heteroaromatic group selected from the group consisting of a furan ring and a thiophene ring, or a cyclohexyl group.

8. A compound according to Claim 1, wherein Y represents a single bond, or a 3- to 6-membered cycloalkylene group or a straight or branched chain alkylene group having 1 to 4 carbon atoms which may have one or two substituents selected from the group consisting of a lower alkenyl group, a lower alkylthio group, a carbamoyl group, a hydroxy group, a sulfamoyl group and a thiocarbamoyl group.

9. A compound according to Claim 1 or 8, wherein Y represents a single bond, or a 3- to 6-membered cycloalkylene group or a straight or branched chain alkylene group having 1 to 4 carbon atoms which may have a substituent selected from the group consisting of a lower alkenyl group, a lower alkylthio group, a carbamoyl group, a hydroxy group, a sulfamoyl group and a thiocarbamoyl group.

10. A method for producing a compound represented by the following general formula [I]: 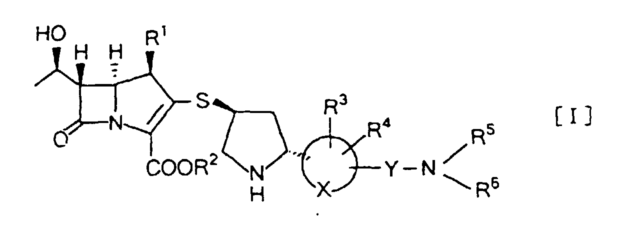 wherein R¹ represents a hydrogen atom or a lower alkyl group; R² represents a hydrogen atom, an ester residue or an alkali metal; R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group; R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group or a lower alkyl group which may have one or two substituents selected from the group consisting of a carbamoyl group, a carboxyl group and a hydroxy group; the group represented by the formula 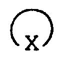 represents an aryl group selected from the group consisting of a benzene ring and a naphthalene ring, a heteroaromatic group selected from the group consisting of a pyrrole ring, a furan ring and a thiophene ring, or a 5- to 8-membered cycloalkyl group; and Y represents a single bond, or a 3- to 6-membered cycloalkylene group or a straight or branched chain alkylene group having 1 to 4 carbon atoms which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group; provided that a case where R³ and R⁴ both represent a hydrogen atom, X is a benzene ring, Y is a methylene group and R⁵ and R⁶ both represent a hydrogen atom is excepted,
characterized by reacting a compound represented by the following general formula [II]: 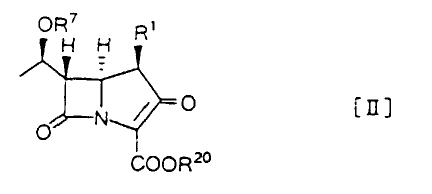 wherein R¹ is as defined above, R⁷ represents a hydrogen atom or a protecting group of a hydroxy group, and R²⁰ represents a hydrogen atom or a protecting group of a carboxyl group, or a reactive derivative of the compound [II] with a compound represented by the following general formula [III]: 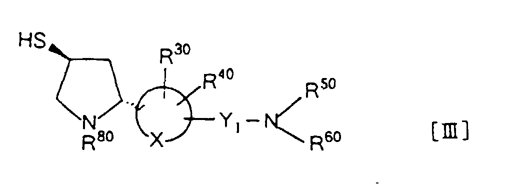 wherein R³⁰ and R⁴⁰, identical or different, represent a hydrogen atom, a halogen atom, a lower alkyl group, a lower alkenyl group, a lower alkynyl group, a lower alkoxy group, a lower alkylthio group, a carbamoyl group, a carboxyl group which may be protected, a cyano group, a hydroxy group which may be protected, a nitro group, a sulfamoyl group, a sulfamoylamino group or an ureido group; R⁵⁰ and R⁶⁰, identical or different, represent a hydrogen atom, a lower alkoxy group, an amino-substituted lower alkyl carbonyl group which may be protected, a sulfamoyl group, a formimidoyl group, a lower alkylimidoyl group, an amidino group, or a lower alkyl group which may have one or two substituents selected from the group consisting of a carbamoyl group, a carboxyl group which may be protected and a hydroxy group which may be protected; the group represented by the formula 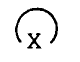 is as defined above; Y₁ represents a single bond, a 3- to 6-membered cycloalkylene group or a straight or branched chain alkylene group having 1 to 4 carbon atoms which may have one or two substituents selected from the group consisting of a halogen atom, a lower alkenyl group, a cyclo(lower alkyl) group, a lower alkoxy group, a lower alkylthio group, a carbamoyl group, a carboxyl group which may be protected, a cyano group, a hydroxy group which may be protected, a nitro group, a sulfamoyl group, a sulfamoylamino group, a thiocarbamoyl group and an ureido group; and R⁸⁰ represents a hydrogen atom or a protecting group of an imino group; provided that a case where R³⁰ and R⁴⁰ both represent a hydrogen atom, the group represented by the formula 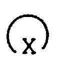 is a benzene ring, Y₁ is a methylene group and R⁵⁰ and R⁶⁰ both represent a hydrogen atom is excepted,
or a salt of the compound [III] to form a compound represented by the following general formula [IV]: 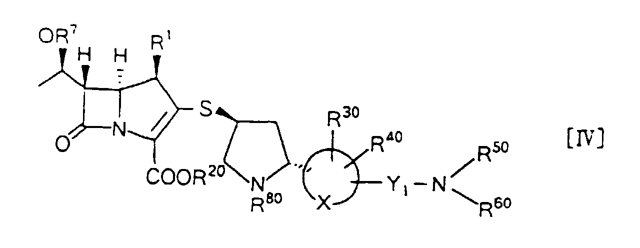 wherein R¹, R⁷, R²⁰, R³⁰, R⁴⁰, R⁵⁰, R⁶⁰, R⁸⁰, the group of the formula 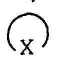 and Y₁ are as defined above, and, if necessary, removing the protecting group from the compound [IV], and/or converting the compound [IV] thus formed into a pharmacologically acceptable salt or a non-toxic ester thereof.

11. An antibacterial agent containing, as an active ingredient thereof, a compound represented by the following general formula [I]: 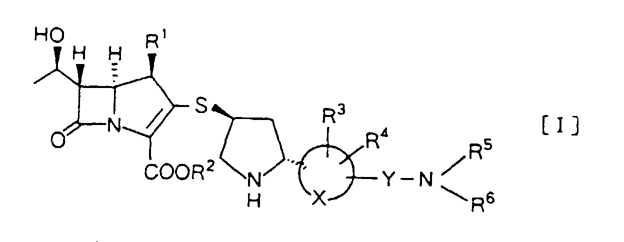 wherein R¹ represents a hydrogen atom or a lower alkyl group; R² represents a hydrogen atom, an ester residue or an alkali metal; R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group; R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxy group;
the group represented by the formula 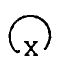 represents an aryl group selected from the group consisting of a benzene ring and a naphthalene ring, a heteroaromatic group selected from the group consisting of a pyrrole group, a furan ring and a thiophene ring, or a 5- to 8-membered cycloalkyl group; Y represents a single bond, or a 3- to 6-membered cycloalkylene group or a straight or branched chain alkylene group having 1 to 4 carbon atoms which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group; provided that a case where R³ and R⁴ both represent a hydrogen atom, the group represented by the formula 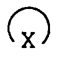 is a benzene ring, Y is a methylene group and R⁵ and R⁶ both represent a hydrogen atom is excepted.

12. A metallo-β-lactamase inhibitor containing, as an active ingredient thereof, a compound represented by the following general formula [I]: 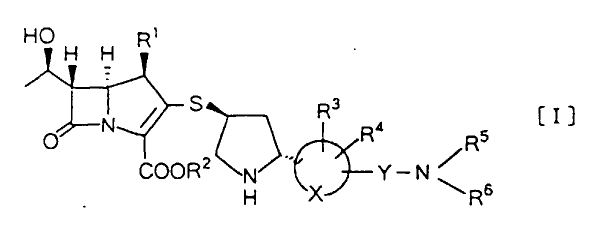 wherein R¹ represents a hydrogen atom or a lower alkyl group; R² represents a hydrogen atom, an ester residue or an alkali metal; R³ and R⁴, identical or different, represent a hydrogen atom, a halogen atom, a lower alkyl group, a lower alkenyl group, a lower alkynyl group, a lower alkoxy group, a lower alkylthio group, a carbamoyl group, a carboxyl group, a cyano group, a hydroxy group, a nitro group, a sulfamoyl group, a sulfamoylamino group or an ureido group; R⁵ and R⁶, identical or different, represent a hydrogen atom, a lower alkoxy group, an amino-substituted lower alkyl carbonyl group, a sulfamoyl group, a formimidoyl group, a lower alkylimidoyl group, an amidino group, or a lower alkyl group which may have one or two substituents selected from the group consisting of a carbamoyl group, a carboxyl group and a hydroxy group;
the group represented by the formula 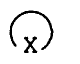 represents an aryl group selected from the group consisting of a benzene ring and a naphthalene ring, a heteroaromatic group selected from the group consisting of a pyrrole group, a furan ring and a thiophene ring, or a 5- to 8-membered cycloalkyl group; Y represents a single bond, or a 3- to 6-membered cycloalkylene group or a straight or branched chain alkylene group having 1 to 4 carbon atoms which may have one or two substituents selected from the group consisting of a halogen atom, a lower alkenyl group, a cyclo(lower alkyl) group, a lower alkoxy group, a lower alkylthio group, a carbamoyl group, a carboxyl group, a cyano group, a hydroxy group, a nitro group, a sulfamoyl group, a sulfamoylamino group, a thiocarbamoyl group and an ureido group; provided that a case where R³ and R⁴ both represent a hydrogen atom, the group represented by the formula 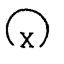 is a benzene ring, Y is a methylene group and R⁵ and R⁶ both represent a hydrogen atom is excepted.
